(19) Europäisches Patentamt
European Patent Office
Office européen des brevets

(11) Veröffentlichungsnummer: **0 018 001**
**B1**

# (12) EUROPÄISCHE PATENTSCHRIFT

(45) Veröffentlichungstag der Patentschrift: **25.05.83**

(51) Int. Cl.³: **C 07 D 501/38**, C 12 Q 1/34

(21) Anmeldenummer: **80102106.4**

(22) Anmeldetag: **18.04.80**

(54) **Chromophore Cephalosporine, Verfahren zu ihrer Herstellung und ihre Verwendung, Zubereitungen, die diese enthalten, sowie ein Verfahren zur Herstellung dieser Zubereitungen.**

(30) Priorität: **24.04.79 DE 2916433**

(43) Veröffentlichungstag der Anmeldung: **29.10.80 Patentblatt 80/22**

(45) Bekanntmachung des Hinweises auf die Patenterteilung: **25.05.83 Patentblatt 83/21**

(84) Benannte Vertragsstaaten: **AT BE CH DE FR GB IT LI NL SE**

(56) Entgegenhaltungen:
**US-A-4 108 504**
**CHEMICAL ABSTRACTS, Band 81, Nr. 1, 8. Juli 1974, Zusammenfassung Nr. 3930m, Seite 322, Spalte 2, COLUMBUS, OHIO (US)**

(73) Patentinhaber: **HOECHST AKTIENGESELLSCHAFT, Postfach 80 03 20, D-6230 Frankfurt/Main 80 (DE)**

(72) Erfinder: **Schindler, Peter, Dr., Reinhardswaldweg 1, D-6082 Mörfelden-Walldorf (DE)**
Erfinder: **Huber, Gerhard, Dr., In den Bleichwiesen 2, D-6233 Kelkheim (Taunus) (DE)**

EP 0 018 001 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Chromophore Cephalosporine, Verfahren zur ihrer Herstellung und ihre Verwendung, Zubereitungen, die diese enthalten, sowie ein Verfahren zur Herstellung dieser Zubereitungen

Der therapeutische Nutzen von β-Lactam-Antibiotika wird eingeschränkt durch das Auftreten β-Lactam-resistenter Bakterien. Die Resistenz hängt ursächlich mit der Bildung von β-Lactamasen (Penicillin-(Cephalosporin)-β-Lactam-Amidohydrolasen, EC 3.5.2.6) zusammen, die die C-N-Bindung des β-Lactamrings von Penicillinen und Cephalosporinen hydrolysieren, was zum Verlust der antibiotischen Eigenschaften dieser Verbindungen führt. Um den gewünschten Therapieeerfolg bei der Behandlung bakterieller Infektionen mit β-Lactam-Antibiotika zu sichern, müssen vor Therapie-Beginn gesicherte Erkenntnisse darüber vorliegen, ob die klinischen Isolate β-Lactamasen produzieren oder nicht. Fällt der β-Lactamase-Nachweis positiv aus, empfiehlt sich entweder der Einsatz von β-Lactamase-resistenten Antibiotika oder aber von anderen klinisch anwendbaren Antibiotika.

Für den Nachweis β-Lactamase-produzierender Keime sind eine Reihe von Techniken beschrieben worden (vgl. J. Pharmacol. 15 (1963), S. 81 – 91). Die genannten Methoden sind jedoch alle nicht völlig befriedigend, da sie, sofern es sich um die für klinisch-diagnostische Zwecke besonders geeigneten colorimetrischen Teste handelt, alle den Zusatz eines Indikators (Jod/Stärke, Hydroxylamin, pH-Indikator) benötigen.

Gegenüber den β-Lactamase-Nachweisen mit Hilfe eines solchen gekoppelten Testansatzes bieten chromogene β-Lactamase-Substrate den Vorteil, daß die Öffnung des β-Lactam-Rings durch diese Enzyme unmittelbar zu einer Farbverschiebung im sichtbaren Teil des Spektrums führt. Ein solches Substrat (Verbindung 87/312) wurde von O'Callaghan et al. beschrieben (Antimicrob. Ag. Chemother. 1 (1972), S. 283 – 288). Nach Angabe der Autoren tritt eine bathochrome Verschiebung der Farbe von Lösungen der Verbindung 87/312 jedoch nicht nur in Gegenwart von β-Lactamasen, sondern in unspezifischer Weise auch nach Zusatz von Serum, Tiergewebe, Eiweiß, Milch, Cystein, Glutathion, Mercaptoäthanol und 2,3-Dimercaptopropanol-1 ein. Dies kann zu einer falschen Beurteilung klinischer Isolate führen.

Gegenstand der Erfindung sind deshalb neue, chromophore Cephalosporine als β-Lactamase-Substrate, die durch einen optisch sehr gut sichtbaren Farbumschlag, beispielsweise von einem intensiven Violett nach Gelb, sowohl für den Nachweis β-Lactamase-produzierender Keime als auch für die Bestimmung der Aktivität isolierter β-Lactamase-produzierender Keime sowie für die Bestimmung der Aktivität isolierter β-Lactamasen sehr gut geeignet sind und die gegen die erwähnten Zusätze stabil sind.

Die erfindungsgemäßen Verbindungen werden von allen untersuchten β-Lactamasen aus gram-negativen Organismen gespalten, insbesondere von den Enzymen aus klinisch interessanten Organismen, z. B. E.coli $R_{TEM}$, Klebsiella aerogenes 1082 E, Pseudomonas aeruginosa 18 SH, Enterobacter cloacae P 99 und Bacteroides fragilis 620. Die erfindungsgemäßen Verbindungen werden ebenso von β-Lactamasen aus grampositiven Organismen, z. B. Staphylococcus aureus R 85, gespalten. Die Verbindungen eignen sich darüber hinaus auch zum Nachweis β-Lactamase-produzierender Keime , d. h. zum Nachweis von β-Lactamasen mit intakten Zellen dieser Organismen.

Der Nachweis der β-Lactamase-Aktivität mit den erfindungsgemäßen Verbindungen erfolgt direkt, d. h. ohne einen gekoppelten Testansatz, da die chromophoren Substituenten, die im oben genannten Zusammenhang Gegenstand der Erfindung sind, synchron mit der Öffnung des β-Lactam-Rings quantitativ eliminiert werden, was unmittelbar zur (für die einzelnen Substituenten spezifischen) Farbverschiebung führt.

Überraschenderweise wurde nun festgestellt, daß die erfindungsgemäßen Verbindungen im Gegensatz zu β-Lactamase-Substraten des Typs 87/312 in wäßrigen Lösungen, die für die Aktivität der β-Lactamasen bzw. für die Lebensfähigkeit von Bakterien geeignet sind, insbesondere aber im neutralen pH-Bereich, außerordentlich beständig sind gegen hydrolytischen Abbau im Temperaturbereich zwischen −180°C und 60°C. Obwohl die Aufbewahrung fertig zubereiteter Lösungen vorteilhafterweise bei −20°C erfolgt, bei der die Lösung nahezu unbegrenzt haltbar ist, können neutral gepufferte Lösungen über längere Zeit bei Kühlschranktemperatur und sogar bei Raumtemperatur aufbewahrt werden, ohne daß ihre Verwendbarkeit beeinträchtigt wird.

Darüber hinaus erwiesen sich die erfindungsgemäßen Verbindungen vollkommen stabil gegenüber dem Einfluß von Serum, Eiweiß, Milch, Cystein, Glutathion, Mercaptoäthanol und 2,3-Dimercaptopropanol-1, (Dimercaprol), wodurch eine weitere unerwartete Überlegenheit gegenüber β-Lactamase-Substraten des Typs 87/312 erzielt werden konnte.

Gegenstand der Erfindung sind somit in 3-Stellung eine chromophore Gruppierung tragende Cephalosporine der allgemeinen Formel I

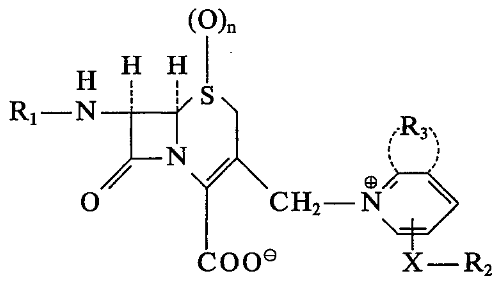

(I)

in der $R_1$ Wasserstoff, Formyl oder einen Rest der Formel

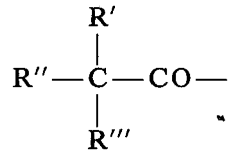

bedeutet, in der R′ für Wasserstoff, für Alkyl mit 1 bis 4 C-Atomen, das substituiert sein kann durch Halogen, Cyano, Hydroxy, Alkoxy mit 1 bis 4 C-Atomen, ω-Carboxy-, ω-Carboxy-ω-amino oder ω-Carboxy-ω-acylamino mit 1 bis 7 C-Atomen im Acylteil, für einen gesättigten oder ein- oder mehrfach ungesättigten carboxyclischen Ring mit 5 bis 7 C-Atomen, für Phenyl oder Phenyloxy, das ein oder zweifach substituiert sein kann durch Alkyl mit 1 bis 4 C-Atomen, Hydroxy, Alkoxy mit 1 bis 4 C-Atomen, Acyloxy mit 1 bis 4 C-Atomen, Halogen, Carboxy, Sulfoxyl, Amino oder Acylamino mit 1 bis 4 C-Atomen, für einen 5- oder 6gliedrigen, heteroaromatischen Ring, der als Heteroatome Stickstoff, Schwefel und/oder Sauerstoff enthalten kann, für einen Thiazolrest der Formel

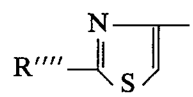

worin
R′′′′ Alkyl mit 1 bis 4 C-Atomen, Amino, Acylamino mit 1 oder 2 C-Atomen, Chlor-, Brom- oder Trifluoracetamido bedeutet, für 3,5-Dichlor-5-pyridon-1-yl, für 2-Methyl-1,3,4-thiadiazol-5-yl-thio oder 2-Amino-1,3,4-thiadiazol-5-yl-thio,
R′′ und R′′′, die gleich oder verschieden sein können, für Wasserstoff, Alkyl mit 1 bis 4 C-Atomen, Hydroxy, Acyloxy mit 1 bis 4 C-Atomen, Alkoxy mit 1 bis 4 C-Atomen, Amino, tert.-Butylamino, tert.-Amylamino, Benzylamino, p-Methoxybenzylamino, Benzhydrylamino, Tritylamino, Phenylethylamino, Formamido, Acetylamino, Chloracetylamino, Bromacetylamino, Benzoylamino, tert.-Butoxycarbonylamino, 2,2,2-Trichlor-ethoxycarbonylamino, 4-Hydroxy-1,5-naphthyridin-3-carbonylamino, 3-Hydroxy-pyridazion 4-carbonylamino, Imidazolin-2-on-1-yl, carbonylamino, (3-Methyl-sulfonyl-imidazolidin-2-on-yl)-carbonylamino, (4-Ethyl-piperazin-2,3-dion-1-yl)-carbonyl-amino, l)-carbonyl-amino, Alkoxycarbonyl mit 1 bis 4 C-Atomen im Alkylteil, Halogen, Cyano, Sulfoxy oder Sulfamoyl stehen oder zusammen Sauerstoff sein können,
X für $-(CH=CH)_m-$ mit m = 1 bis 4 oder $-N=N-$ oder Kombinationen dieser Gruppen steht, $R^2$ für Phenyl steht, das substituiert sein kann durch Hydroxy, Alkoxy mit 1 bis 4 C-Atomen, Acyloxy mit 1 bis 4 C-Atomen oder durch

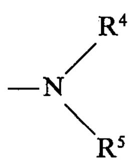

worin
$R^4$ und $R^5$ gleich oder verschieden sein können und Wasserstoff, Alkyl mit 1 bis 4 C-Atomen, Cyanoalkyl mit 1 bis 4 C-Atomen im Alkylteil, Carboxyalkyl mit 1 bis 4 C-Atomen im Alkylteil, Alkoxycarbonylalkyl mit jeweils 1 bis 4 C-Atomen in den Alkylteilen, Hydroxyalkyl mit 1 bis 4 C-Atomen, Acyloxyalkyl mit 1 bis 4 C-Atomen im Acyl- und Alkylteil, Carbamoylalkyl mit 1 bis 4 C-Atomen im Alkylteil, Sulfoxyalkyl mit 1 bis 4 C-Atomen oder Halogenalkyl mit 1 bis 4 C-Atomen,
$R^3$ für zwei Wasserstoffatome sowie für einen angegliederten Benzolring oder 5- oder 6gliedrigen heteroaromatischen Ring mit 1 bis 2 Stickstoffatomen und/oder einem Schwefelatom steht und
n die Bedeutung von 0, 1 oder 2 besitzt.

Der Rest $-X-R_2-$ kann in α,β oder γ-Stellung des Pyridiniumringes stehen.

Im Sinne der Erfindung besonders bevorzugt sind Verbindungen der allgemeinen Formel II

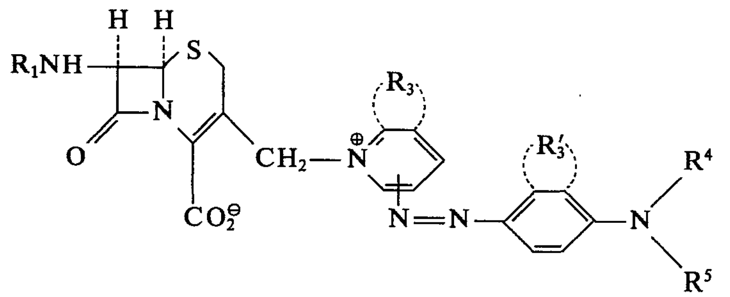

(II)

worin
$R_1$ und $R_3$ die vorgenannten Bedeutungen haben,
$R_3'$ eine der für $R_3$ angegebenen Bedeutung besitzt und
$R_4$ und $R_5$ die gleich oder verschieden sein können, für Wasserstoff oder Alkyl mit 1–4 C-Atomen stehen, das gegebenenfalls substituiert sein kann mit Halogen, Hydroxy, Acyloxy mit 1–4 C-Atomen, Alkoxy mit 1–4 C-Atomen, Alkoxycarbonyl mit 1–4 C-Atomen im Alkoxyteil, Carboxy, Cyano, Carbamoyl oder Sulfoxy.

Die Verknüpfung der Aminophenyldiazogruppe mit dem Pyridiniumkern führt je nach Stellung zu den unterschiedlichen erfindungsgemäßen Verbindungen IIa, IIb und IIc:

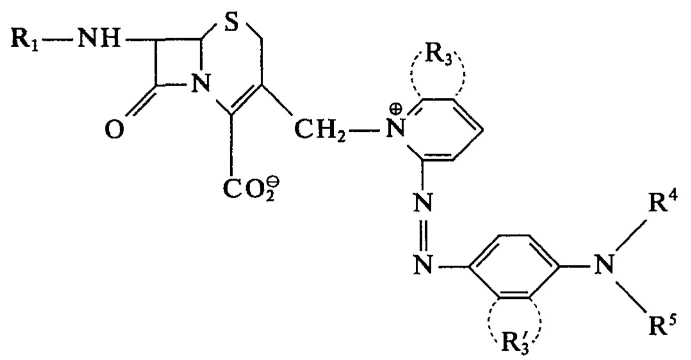

(IIa)

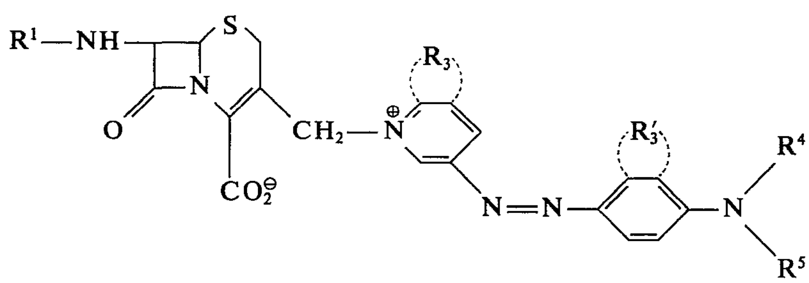

(IIb)

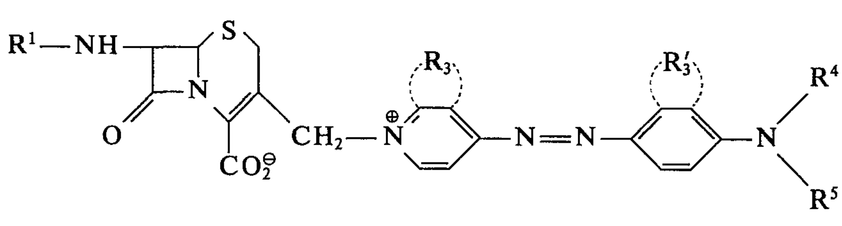

(IIc)

Bei den erfindungsgemäßen Verbindungen haben die Substituenten insbesondere die folgenden Bedeutungen:

R′ kann stehen für Wasserstoff, für Alkyl mit 1–4 C-Atomen, vorzugsweise Methyl oder Äthyl, für substituiertes Alkyl mit 1–4 C-Atomen, wie beispielsweise für Halogenalkyl mit 1–4 C-Atomen, vorzugsweise Chlormethyl, Brommethyl, Chloräthyl oder Bromäthyl, für Cyanoalkyl mit 1–4 C-Atomen im Alkylteil, vorzugsweise Cyanomethyl oder Cyanoäthyl, für Hydroxyalkyl mit 1–4 C-Atomen, vorzugsweise Hydroxymethyl oder Hydroxyäthyl, für Alkoxyäthyl mit 1–4 C-Atomen sowohl im Alkoxy- als auch im Alkylteil, vorzugsweise Methoxymethyl, Äthoxymethyl oder Methoxyäthyl, für $\omega$-Carboxy-$\omega$-amino-alkyl mit 1–4 C-Atomen im Alkylteil, vorzugsweise $\omega$-Carboxy-$\omega$-amino-propyl, für $\omega$-Carboxy-$\omega$-acylaminoalkyl mit 1–4 C-Atomen im Alkylteil und 1–7 C-Atomen im Acylteil, wie

vorzugsweise ω-Carboxy-ω-benzoylamino-propyl, für ω-Carboxyalkyl mit 1–4 C-Atomen im Alkylteil, vorzugsweise Carboxymethyl oder Carboxyäthyl, einen gegebenenfalls substituierten, gesättigten oder ein- oder mehrfach ungesättigten, 5- bis 7gliedrigen carbocyclischen Ring, wie Cycloalkyl, vorzugsweise Cyclohexyl, Cycloalkenyl, vorzugsweise Cyclohexenyl, Cycloalkadienyl, vorzugsweise Cyclohexadienyl, gegebenenfalls substituiertes Aryl oder Aryloxy der Formeln

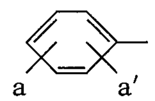 und 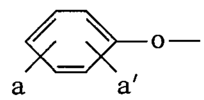

in denen a und a' gleich oder verschieden sein können und Wasserstoff, $C_1-C_4$-Alkyl, vorzugsweise Methyl oder Äthyl, Hydroxy, $C_1-C_4$-Alkoxy, vorzugsweise Methoxy oder Äthoxy, $C_1-C_4$-Acyloxy, vorzugsweise Acetoxy, Halogen, vorzugsweise Fluor und Chlor, Carboxy, Sulfoxy, Amino, $C_1-C_4$-Acylamino, vorzugsweise Acetamino, bedeuten kann.

R' kann weiterhin stehen für gegebenenfalls substituiertes, 5- bis 6gliedriges Heteroaryl, das als Heteroatome Stickstoff, Schwefel oder Sauerstoff enthalten kann, wie z. B. 2-Pyridon-1-yl, 4-Pyridon-1-yl, 3,5-Dichlor-5-pyridon-1-yl, 2-Thienyl, 3-Thienyl, 2- und 3-Furyl, 1-Tetrazolyl, vorzugsweise aber 2-Furyl, 2-Thienyl, 1-Tetrazolyl oder ein Thiazolyl der Formel

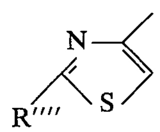

in der R'''' vorzugsweise Alkyl mit 1 bis 4 C-Atomen, insbesondere Methyl oder Äthyl, Amino, gegebenenfalls substituiertes Acylamino, wie insbesondere Formylamino, Acetylamino, Chloracetylamin, Bromacetylamino oder Trifluoracetylamino sein kann. R' kann auch stehen für einen gegebenenfalls substituierten Heteroarylthiorest, wie vorzugsweise 2-Methyl-1,3,4-thiadiazol-5-yl-thio oder 2-Amino-1,3,4-thiadiazol-5-yl-thio.

R'' und R''', die gleich oder verschieden sein können, können die Bedeutung besitzen von Wasserstoff, von Alkyl mit 1–4 C-Atomen, vorzugsweise Methyl, von Hydroxy, von gegebenenfalls substituiertem Acyloxy mit 1 bis 4 C-Atomen im Acylteil, wie z. B. Formyloxy, Acetoxy, Propionyloxy, von gegebenenfalls substituiertem Alkoxy mit 1 bis 4 C-Atomen im Alkylteil, wie z. B. Methoxy, Äthoxy, Propoxy, von Amino, von gegebenenfalls substituiertem Alkylamino, wie vorzugsweise tert.-Butylamino, tert.-Amylamino, Benzylamino, p-Methoxybenzylamino, Benzhydrylamino, Tritylamino, Phenyläthylamino, von gegebenenfalls substituiertem Acylamino, wie z. B. Formylamino, Acetylamino, Chloracetylamino, Bromacetylamino, Benzoylamino, tert.-Butoxycarbonylamino, 2,2,2-Trichloräthoxycarbonylamino, 4-Hydroxy-1,5-naphthyridin-3-carbonylamino, 3-Hydroxy-pyridazin-4-carbonylamino, Imidazolidin-2-on-1-yl-carbonylamino, (3-Methyl-sulfonyl-imidazolidin-2-on-1-yl)-carbonylamino, (4-Äthyl-piperazin-2,3-dion-1-yl)-carbonylamino, von Alkoxycarbonyl mit 1 bis 4 C-Atomen im Alkylteil, wie z. B. Methoxycarbonyl, Äthoxycarbonyl, von Halogen, vorzugsweise Chlor und Brom, von Cyano, von Sulfoxy von Sulfamoyl, oder zusammen von Sauerstoff.

X steht bevorzugt für $-N=N-$, $R_2$ kann stehen für Phenyl, das gegebenenfalls substituiert sein kann durch Hydroxy, durch Alkoxy mit 1–4 C-Atomen, wie vorzugsweise Methoxy und Äthoxy, durch Acyloxy mit 1–4 C-Atomen, wie vorzugsweise Acetoxy und Propionyloxy, insbesondere jedoch durch eine Gruppe der Formel

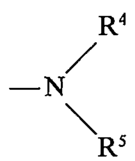

in der $R_4$ und $R_5$ gleich oder verschieden sein können und Wasserstoff, Alkyl mit 1–4 C-Atomen, vorzugsweise Methyl und Äthyl, Cyanoalkyl mit 1–4 C-Atomen im Alkylteil, wie vorzugsweise Cyanoäthyl, Carboxyalkyl mit 1–4 C-Atomen im Alkylteil wie vorzugsweise Carboxyäthyl, Carbamoylalkyl mit jeweils 1–4 C-Atomen in den Alkylteilen wie vorzugsweise Äthoxycarbonyläthyl, Hydroxyalkyl mit 1–4 C-Atomen, wie vorzugsweise Hydroxyäthyl oder Acyloxyalkyl mit jeweils 1–4 C-Atomen im Acyl- und Alkylteil wie vorzugsweise Acetoxyäthyl, Carbamoylalkyl mit 1–4 C-Atomen im Alkylteil wie vorzugsweise Carbamoyläthyl, Sulfoxyalkyl mit 1–4 C-Atomen, vorzugsweise Sulfoxyäthyl, Halogenalkyl mit 1–4 C-Atomen wie vorzugsweise Chloräthyl bedeuten.

$R_3$ und $R_3'$ können gleich oder verschieden sein und können stehen für zwei Wasserstoffatome, einen angegliederten aromatischen Ring wie vorzugsweise Benzo oder einen angegliederten heteroaromatischen 5- oder 6gliedrigen Ring mit 1 oder 2 Heteroatomen, insbesondere Stickstoff und/oder Schwefel wie vorzugsweise Pyrido, Thieno, Pyrimidino, Thiazolo.

Gegenstand der Erfindung ist weiterhin ein Verfahren zur Herstellung der Cephalosporine der allgemeinen Formel I, das dadurch gekennzeichnet ist, daß man eine Verbindung der Formel III

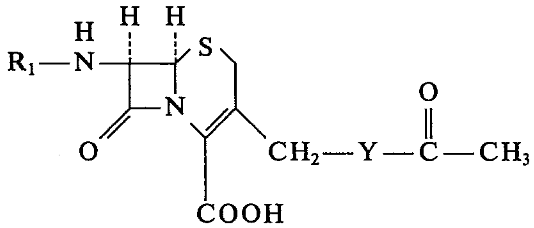

(III)

in der $R_1$ die vorgenannte Bedeutung hat und Y für Sauerstoff oder Schwefel steht, mit einer Verbindung der Formel IV

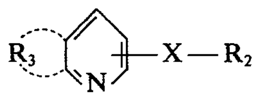

(IV)

in der $R_2$, $R_3$ und X die obengenannten Bedeutungen haben, umsetzt.

Sollen die bevorzugten Verbindungen der allgemeinen Formel II erhalten werden, so bringt man Verbindungen der Formel III mit Verbindungen der Formel IVa

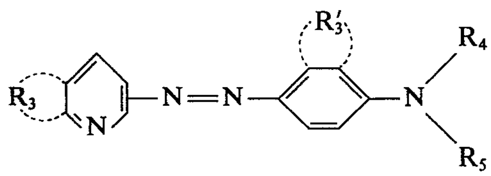

(IV a)

zur Reaktion zu denen $R_3$, $R'_3$, $R_4$ und $R_5$ die oben angegebenen Bedeutungen besitzen.

Die erfindungsgemäß ganz besonders bevorzugte 7-(Thienyl-2-acetamido)-3[2-(4-N,N-dimethyl-amino-phenyl-azo)-pyridino-methyl]-3-cephem-4-carboxylat der Formel

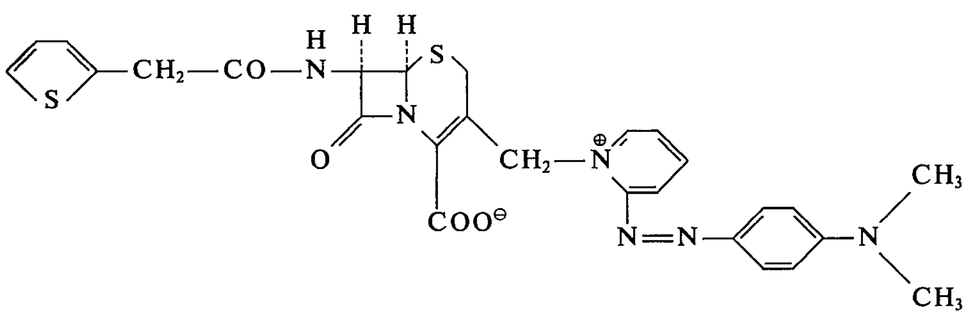

die auch als PADAC (»Pyridin-azo-dimethylanilino-Cephalotin«) bezeichnet wird, kann so durch Umsetzung der Verbindung PADA (trans-Pyridin-azo-p-dimethyl-anilin) der Formel

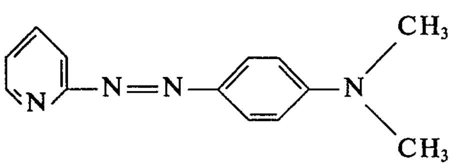

mit Cephalotin der Formel

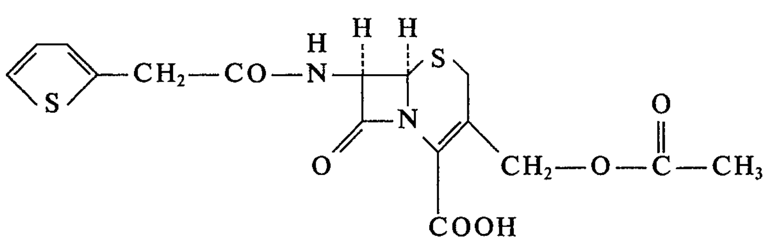

gewonnen werden.

Die zur Durchführung des erfindungsgemäßen Verfahrens erforderlichen Ausgangssubstanzen sind literaturbekannt oder können nach literaturbekannten Verfahren hergestellt werden.

Die Umsetzung von Verbindungen der Formel III mit Verbindungen der Formel IV, insbesondere IVa, wird zweckmäßigerweise im wäßrigen Medium durchgeführt, wobei zur Verbesserung der Löslichkeit mit Wasser mischbare Lösungsmittel wie z. B. Methanol, Äthanol, Isopropanol, Acetonitril oder Aceton zugefügt werden. Die Reaktionskomponenten können im beliebigen Verhältnis, vorteilhaft jedoch im molaren Verhältnis, umgesetzt werden. Die Reaktion wird bei Temperaturen zwischen etwa 20 und 80°C durchgeführt, vorteilhafterweise zwischen 55 und 65°C. Während der Reaktion wird der pH-Wert der Reaktionslösung unter Rühren durch Zugabe beispielsweise Natriumhydroxyd- oder gesättigter Natriumbikarbonat-Lösung auf etwa 5 bis 8 gehalten, vorzugsweise zwischen 6,5 und 7,5.

Zur Verbesserung der Umsetzung kann zum Reaktionsmedium Kaliumjodid oder Kaliumrhodanid (beispielsweise etwa 5 bis 20, vorzugsweise 10 bis 15 Mol pro Mol des eingesetzten Cephalosporins) zugesetzt werden.

Nach beendeter Umsetzung werden die erfindungsmäßen Reaktionsprodukte isoliert. Hierzu kann nach Abdestillieren des mit Wasser mischbaren Lösungsmittels, z. B. Aceton, die wäßrige Phase zur Entfernung der nicht umgesetzten Verbindungen der Formeln IV oder IVa, z. B. PADA, mit Essigester oder anderen geeigneten Lösungsmitteln extrahiert werden. Die Reaktionsprodukte können aus der wäßrigen Phase durch Ausfällen bei einem pH-Wert von etwa 2 bis 4, vorzugsweise bei pH 3, oder durch Extraktion mit Methylenchlorid oder mit geeigneten organischen Lösungsmitteln bei gleichem pH gewonnen werden. Nach Einengen des Extraktes im Vakuum kann man die Reaktionsprodukte durch Zusatz eines organischen Lösungsmittels, in welchem diese schwer löslich sind, z. B. Diäthyläther oder Petroläther, ausfällen.

In einer anderen Ausführungsform des Verfahrens können die Verbindungen der allgemeinen Formel I so hergestellt werden, daß man Verbindungen der Formel III, worin $R_1$ Wasserstoff bedeutet, mit einer Verbindung der Formel IV in der beschriebenen Weise umsetzt und anschließend die gewonnene chromophore Verbindung der allgemeinen Formel I, in der $R_1$ für Wasserstoff steht, mit einer Carbonsäure der allgemeinen Formel V

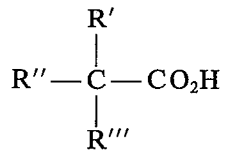

(V)

in der R', R'' und R''' die vorgenannten Bedeutungen haben oder einem aktivierten Derivat dieser Carbonsäure in an sich bekannter Weise umsetzt.

Als aktivierte Derivate der Carbonsäure der allgemeinen Formel V eignen sich insbesondere die Halogenide, vorzugsweise Chloride und Bromide, ferner die Anhydride und gemischten Anhydride, die Azide und aktivierten Ester, vorzugsweise die mit p-Nitrophenol, 2,4-Dinitrophenol, Methylencyanhydrin, N-Hydroxysuccinimid und N-Hydroxyphthalimid, besonders bevorzugt mit 1-Hydroxybenzotriazol und 6-Chlor-1-H-hydroxybenzotriazol. Als gemischte Anhydride eigenen sich besonders solche mit niederen Alkansäuren, z. B. mit Essigsäure und besonders bevorzugt nit substituierten Essigsäuren, wie z. B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel V mit Chlorameisensäurebenzylester, -p-nitrobenzylester, -iso-butylester, -äthylester oder -allylester gewinnt. Die aktivierten Derivate können als isolierte Substanzen, aber auch in situ umgesetzt werden. Allgemein erfolgt die Umsetzung der Cephemderivate der Formel I, worin $R_1$ für Wasserstoff steht, mit der Carbonsäure der Formel V oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z. B. Diäthyläther, Diisopropyläther und vorzugsweise Tetrahydrofuran und Dioxan; Ketone, wie vorzugsweise Aceton und Butanon; Amide, wie vorzugsweise Dimethylformamid und Dimethylacetamid oder Wasser. Es kann sich auch als vorteilhaft erweisen, Gemische der genannten Lösungsmittel zu verwenden. Dies ist oftmals der Fall, wenn die Cephemverbindung der Formel I, worin $R_1$ für Wasserstoff steht, mit einem in situ erzeugten aktivierten Derivat der Carbonsäure der Formel V umgesetzt wird.

Die Umsetzung von Cephemverbindungen der Formel I, worin $R_1$ für Wasserstoff steht, mit Carbonsäuren der Formel V bzw. deren aktivierten Derivaten kann in einem Temperaturbereich von etwa −50 bis etwa +80°C, vorzugsweise zwischen −20 und +50°C, besonders bevorzugt jedoch zwischen −20°C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Umsetzung von aktivierten Derivaten der Carbonsäuren der Formel V mit Cephemverbindungen der Formel I, worin $R_1$ für Wasserstoff steht, wird vorzugsweise in alkalischem Milieu oberhalb pH 7

vorgenommen. Man setzt hierfür dem Reaktionsgemisch eine Base zu, wie vorzugsweise Kalium- oder Natriumcarbonat, Kalium- oder Natriumbikarbonat, Kalium- oder Natriumhydroxyd, Pyridin oder ein Trialkylamin, wie z. B. Triäthylamin, N-Methylmorpholin, Äthyldiisopropylamin oder Kalium-tert.-butylat.

Die Reindarstellung der erfindungsgemäß erhaltenen Cephalosporine kann in an sich bekannter Weise durch Chromatographie an Kieselgel, Cellulose, Sephadex®-G-10, G-25 oder LH-20, Adsorptionsharzen wie Amberlite®XAD-2 und XAD-4 oder Diaion HP-20 durchgeführt werden.

Die besonderes bevorzugte, vorstehend bereits erwähnte Verbindung PADAC besitzt eine intensive violette Färbung ($\lambda$max 566 nm), die nach Reaktion mit $\beta$-Lactamasen, z. B. einer $\beta$-Lactamase aus Enterobacter cloacae P 99 in eine Gelbfärbung umschlägt ($\lambda$ max 465 nm). Eine analoge Reaktion erfolgt mit anderen $\beta$-Lactamasen, z. B. aus Escherichia coli $R_{TEM}$, Klebsiella aerogenes 1082 E, Enterobacter cloacae P 99, Pseudomonas aeruginosa 18 SH, Bacteroides fragilis 620 und Staph. aureus R 85. Andere erfindungsgemäße chromophore Cephalosporine ergeben bei der Umsetzung mit $\beta$-Lactamasen entsprechende Farbumschläge.

Die erfindungsgemäßen Verbindungen werden zum Nachweis von $\beta$-Lactamasen verwendet, indem z. B. neutral gepufferte wäßrige Lösungen der chromophoren Cephalosporine direkt mit isolierten $\beta$-Lactamasen oder aber mit Zellsuspensionen von $\beta$-Lactamase-produzierenden Keimen versetzt werden, wobei bei geeigneter Wahl der Cephalosporinkonzentration der für die einzelnen Verbindungen charakteristische Farbumschlag beobachtet wird. Der $\beta$-Lactamase-Nachweis läßt sich auch in der Form durchführen, daß die erfindungsgemäßen Verbindungen statt in einer Pufferlösung zunächst in heißem Agar gelöst werden, der alle für das Wachstum von Bakterien notwendigen Nährstoffe enthält. Auf die damit in üblicher Weise hergestellten Testschalen können die entsprechend verdünnten, zu untersuchenden klinischen Proben direkt aufgebracht werden. Nach der Bebrütung der Testschalen bei Temperaturen, die sich nach den spezifischen Erfordernissen der zu untersuchenden Keime richtet, wird in der Umgebung der $\beta$-Lactamase-produzierenden Bakterien derselbe Farbumschlag beobachtet. Alternativ können anderweitig gewonnene klinische Isolate in Form eines Kolonie-Abstrichs auf angefeuchtete Teststreifen aus Papier oder einem anderen geeigneten Träger-Material wie z. B. saugfähige Kunststoffe, Dextrane oder andere natürliche Polymere, ausgestrichen werden, wie vorher mit geeigneten, vorzugsweise wäßrigen Lösungen der erfindungsgemäßen Verbindungen getränkt wurden. Auch hier zeigen $\beta$-Lactamase-positive Proben innerhalb kurzer Zeit den charakteristischen Farbumschlag.

Erfindungsgemäß lassen sich außer den in den Ausführungsbeispielen genannten Verbindungen beispielsweise die in der nachfolgenden Tabelle aufgeführten Verbindungen II herstellen.

| Formel | $R_1$ | $R_3$ | $R_3'$ | $R_4$ | $R_5$ |
|---|---|---|---|---|---|
| IIa | $CH_3CO$ | H, H | H, H | $CH_3$ | $CH_3$ |
| IIa | $C_3H_7CO$ | H, H | H, H | $CH_3$ | $CH_3$ |
| IIa | $CH_3OCH_2CO$ | H, H | H, H | $CH_3$ | $CH_3$ |
| IIa | $CH_3OCH_2CO$ | H, H | H, H | $C_2H_5$ | $C_2H_4CO_2H$ |
| IIa | $NCCH_2CO$ | H, H | H, H | $-CH_2CH_2OCOCH_3$ | $CH_2CH_2OCOCH_3$ |
| IIa | $NCCH_2-S-CH_2CO$ | H, H | H, H | $CH_3$ | $CH_3$ |
| IIa | $NCCH_2-S-CH_2CO$ | H, H | H, H | $CH_3$ | $CH_2CH_2CN$ |
| IIa | HO— 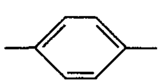 —$CH_2CO$— | H, H | H, H | $CH_3$ | $CH_3$ |
| IIa | 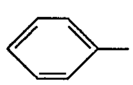 —$CH_2CO$— | H, H | H, H | $C_2H_5$ | $CH_2CH_2CO_2H$ |
| IIb | 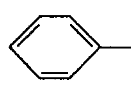 —$CH_2CO$— | H, H | H, H | $C_2H_5$ | $CH_2CH_2CO_2H$ |
| IIb | 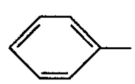 —$CH_2CO$— | H, H | H, H | $C_2H_5$ | $C_2H_5$ |

Fortsetzung

| Formel | $R_1$ | $R_3$ | $R_3'$ | $R_4$ | $R_5$ |
|---|---|---|---|---|---|
| IIa | 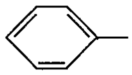 $-CH_2CO-$ | H, H | H, H | $CH_3$ | $CH_2CH_2CN$ |
| IIb | 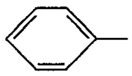 $-CH_2CO-$ | H, H | 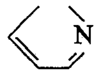 | H | $CH_2CH_2CO_2H$ |
| IIc | 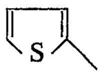 $CH_2-CO-$ | H, H | H, H | $CH_3$ | $CH_3$ |
| IIc | 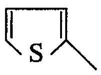 $CH_2-CO-$ | H, H | H, H | $C_2H_5$ | $C_2H_5$ |
| IIc | 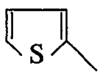 $CH_2-CO-$ | H, H | H, H | $-CH_2CH_2OCOCH_3$ | $CH_2CH_2OCOCH_3$ |
| IIa | 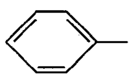 $-OCH_2CO-$ | H, H | H, H | $C_2H_5$ | $C_2H_5$ |
| IIa | 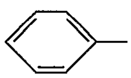 $-OCH_2CO-$ | H, H | H, H | $CH_3$ | $CH_2CH_2CN$ |
| IIa | 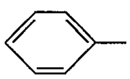 $-OCH_2CO-$ | H, H | H, H | $CH_2CH_2CN$ | $CH_2CH_2OCOCH_3$ |
| IIb | 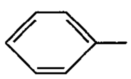 $-OCH_2CO$ | H, H | H | $C_2H_5$ | $C_2H_5$ |
| IIb | 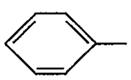 $-OCH_2CO$ | H, H | H | $C_2H_5$ | $CH_2CH_2CO_2H$ |
| IIb | 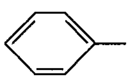 $-OCH_2CO$ | H, H | 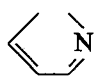 | H | $C_2H_4CO_2H$ |
| IIc | 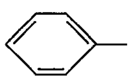 $-OCH_2CO$ | H, H | H, H | $CH_3$ | $CH_3$ |
| IIc | 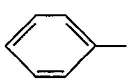 $-OCH_2CO$ | H, H | H, H | $C_2H_5$ | $C_2H_5$ |
| IIa | 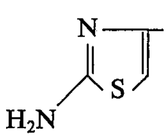 $-CH_2-CO-$ | H, H | H, H | $CH_3$ | $CH_3$ |
| IIa | 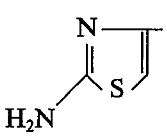 $-CH_2-CO-$ | H, H | H, H | $C_2H_5$ | $C_2H_5$ |
| IIa | 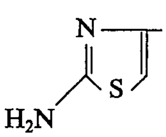 $-CH_2-CO-$ | H, H | H, H | $C_2H_4OCOCH_3$ | $C_2H_4OCOCH_3$ |

Fortsetzung

| Formel | $R_1$ | $R_3$ | $R_3'$ | $R_4$ | $R_5$ |
|---|---|---|---|---|---|
| II b | 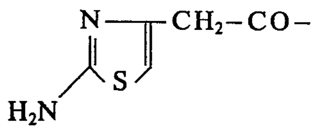 | H, H | H, H | $CH_3$ | $CH_3$ |
| II b | 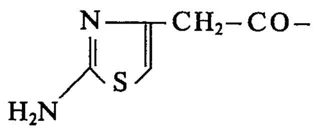 | H, H | H, H | $C_2H_5$ | $C_2H_4CO_2H$ |
| II c | 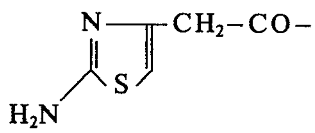 | H, H | H, H | $CH_3$ | $CH_3$ |
| II a | 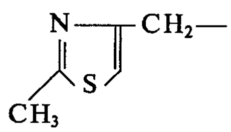 | H, H | H, H | $CH_3$ | $CH_3$ |
| II a | 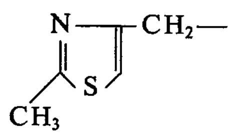 | H, H | H, H | $CH_2CH_2OCOCH_3$ | $CH_2CH_2CN$ |
| II a | 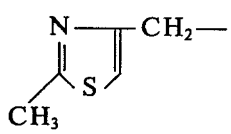 | H, H | H, H | $CH_3$ | $CH_2CH_2CN$ |
| II b | 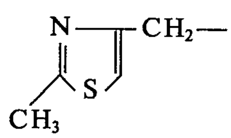 | H, H | H, H | $C_2H_5$ | $C_2H_5$ |
| II b | 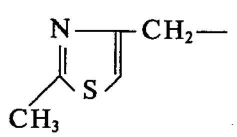 | H, H | H, H | $C_2H_5$ | $C_2H_4CO_2H$ |
| II b | 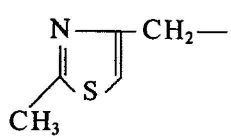 | H, H | H, H | $C_2H_5$ | $C_2H_4CN$ |
| II a | 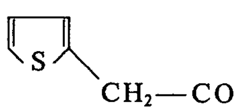 | H, H | H, H | $C_2H_5$ | $C_2H_4—OSO_3H$ |
| II b | 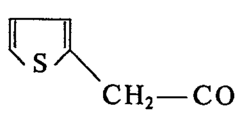 | H, H | H, H | $C_2H_5$ | $C_2H_4—OSO_3H$ |
| II a | 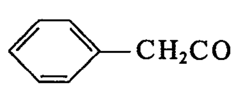 | H, H | H, H | $C_2H_5$ | $C_2H_4—OSO_3H$ |

Fortsetzung

| Formel | $R_1$ | $R_3$ | $R_3'$ | $R_4$ | $R_5$ |
|---|---|---|---|---|---|
| IIb | 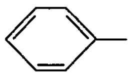—$CH_2CO$ | H,H | H,H | $C_2H_5$ | $C_2H_4$—$OSO_3H$ |
| IIa | 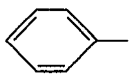—$OCH_2CO$ | H,H | H,H | $C_2H_5$ | $C_2H_4$—$OSO_3H$ |
| IIb | 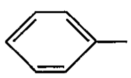—$OCH_2CO$ | H,H | H,H | $C_2H_5$ | $C_2H_4$—$OSO_3H$ |
| IIa | 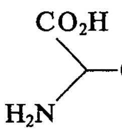—$(CH_2)_3$—CO | H,H | H,H | $C_2H_5$ | $C_2H_4$—$OSO_3H$ |
| IIb | 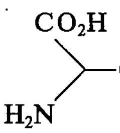—$(CH_2)_3$—CO | H,H | H,H | $C_2H_5$ | $C_2H_4$—$OSO_3H$ |
| IIa | 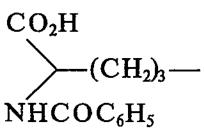—$(CH_2)_3$—CO | H,H | H,H | $C_2H_5$ | $C_2H_4$—$OSO_3H$ |
| IIb | 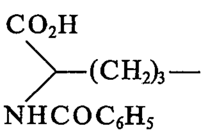—$(CH_2)_3$—CO | H,H | H,H | $C_2H_5$ | $C_2H_4$—$OSO_3H$ |

Die Herstellung und Anwendung der erfindungsgemäßen chromophoren Cephalosporine wird durch folgende Beispiele erläutert:

### Beispiel 1

7-(Thienyl-2-acetamido)-3[2-(4-N,N-dimethylamino-phenylazo)-pyridino-methyl]-3-cephem-4-carboxylat (PADAC)

In einem 1-l-Dreihalsrundkolben mit Rührer, Thermometer und pH-Elektrode wird eine Lösung von 2,26 g (10 mMol) trans-Pyridin-2-azo-4'-dimethylanilin (PADA) in 250 ml Aceton im Wasserbad auf 55—60°C erwärmt. Zu der Lösung wird eine Auflösung von 4,0 g (10 mMol) Cephalotin Na-Salz in 100 ml Wasser gegeben. Nach Einstellung eines pH-Wertes von 7,3—7,5 mit gesättigter Natriumbikarbonatlösung wird die Reaktion im Dünnschichtchromatogramm verfolgt (Kieselgel »Merck« F 254, System I: Äthylacetat/Isopropanol/Wasser 20 : 15 : 10). Das gewünschte Endprodukt erscheint als violette Bande mit $R_f$ 0,54.

Nach Beendigung der Umsetzung wird das Aceton im Vakuum abdestilliert und der wäßrige Rückstand auf pH 8 gestellt. Ungelöste Substanz (unumgesetztes PADA) wird abgetrennt und die Wasserphase mehrmals zur Entfernung der restlichen PADA mit Äthylacetat extrahiert.

Die Wasserphase wird mit verdünnter Salzsäure auf pH 5,7 eingestellt und mehrmals mit je 1 Vol. Methylenchlorid extrahiert. Die vereinigten Methylenchloridphasen werden im Vakuum auf 100 ml eingeengt und PADAC durch Zusatz von 300 ml Diäthyläther ausgefällt. Der violette Niederschlag wird über eine Fritte abgesaugt, mit wenig Äther gewaschen und getrocknet. Ausbeute 1,01 g rohes PADAC als dunkelviolettes Pulver.

0,5 g des rohen PADAC werden in 20 ml Wasser aufgeschlämmt. Die Suspension wird unter Rühren und Kühlung so lange tropfenweise mit 0,1 n NaOH versetzt, bis ein konstanter pH-Wert von 7 erreicht ist. Die Lösung ergibt nach Gefriertrocknung 0,5 g leicht wasserlösliches Natriumsalz des PADAC.

Beispiel 2

7-(Thienyl-2-acetamido)-3-[2-(4-N,N-dimethylamino-phenylazo)-pyridino-methyl]-3-cephem-4-carboxylat (PADAC)

In einem 3-l-Vierhalskolben, der mit Rührer, Thermometer und pH-Elektrode ausgestattet ist, werden 40 g Cephalotin-Natriumsalz und 240 g Kaliumjodid in 1 l Wasser gelöst. Unabhängig davon werden 22,6 g PADA in 500 ml Aceton gelöst und ebenfalls in den Kolben gegeben. Der pH-Wert wird auf 6,5 eingestellt und der Kolben im Wasserbad auf 60°C erwärmt (Thermostat). Während der Reaktion wird der pH-Wert mit gesättigter Natriumbikarbonat-Lösung auf 6,5 eingehalten. Nach 5 Stunden wird das Aceton aus der Reaktionslösung abgezogen und aus der wäßrigen Phase ausgefallenes, unumgesetztes PADA durch Filtration abgetrennt. Die wäßrige Phase wird nach Einstellen auf pH 8 mit Natronlauge dreimal mit je 800 ml Äthylacetat extrahiert. Der wäßrige Rückstand wird mit n-Schwefelsäure auf pH 3 gestellt und das dabei ausgefällte PADAC abfiltriert, mit wenig Wasser gewaschen und im Hochvakuum getrocknet. 11,8 g violettes Pulver.

Aus der Mutterlauge kann weiteres PADAC geringerer Reinheit durch zweimalige Extraktion mit je 1 l Methylenchlorid gewonnen werden. Das Produkt wird nach Eindampfen des Methylenchlorids auf 200 ml mit 600 ml Diäthyläther ausgefällt, abgesaugt, mit Äther gewaschen und getrocknet. 5,2 g rohes PADAC.

1 g des rohen PADAC wird in 20 ml eines Gemisches von Äthylacetat, Isopropanol und Wasser im Verhältnis 4 : 3 : 2 gelöst und auf eine Säule von 110 g Kieselgel »Merck« 60, Korngröße 0,063 bis 0,2 mm, im gleichen Lösungsmittelsystem eingeschlämmt, gegeben. Nach Entwicklung mit diesem System wird nach Abtrennen eines gelb-roten Vorlaufs eine violette Zone eluiert, die das PADAC enthält. Nach Eindampfen der aktiven Fraktion zur wäßrigen Phase wird gefriergetrocknet. Ausbeute 300 mg reines PADAC. $\lambda$ max 566 nm (5% Acetonitril) ($\varepsilon$=42 200), nach Zusatz von $\beta$-Lactamase verschwindend.

IR: 3400, 1768 ($\beta$-Lactam), 1666, 1600, 1562, 1540, 1485, 1390, 1350, 1330, 1300, 1269, 1225, 1150, 1105, 932, 910, 837, 785, 700 $cm^{-1}$.

NMR, $(CD_3)_2SO$: 9,0 (d, 1H), 6,5 – 8,0 (m), 5,5 (m), 5,0 (s), 3,7 (s), 3,1 (s) ppm.

Beispiel 3

7-(Thienyl-2-acetamido)-3-[2-(4-N-methyl-N-cyanoäthylamino-phenylazo)-pyridino-methyl]-3-cephem-4-carboxylat

Eine Lösung von 80 mg Cephalotin Na-Salz in 2 ml Wasser wird mit einer Lösung von 45 mg Pyridin-2-azo-4'-N-methyl-4'-N-cyanoäthylanilin (VI) in 1 ml Aceton gemischt. Die Lösung wird auf pH 6,5 gestellt und im Wasserbad auf 60°C erwärmt. Der Verlauf der Umsetzung wird dünnschichtchromatographisch unter Anwendung von System I (vgl. Beispiel 1) verfolgt. Nach 5 Stunden ist die Umsetzung beendet. Der Ansatz wird mit Äthylacetat extrahiert, die wäßrige Phase mit n-Schwefelsäure auf pH 3 gestellt und mit Methylenchlorid extrahiert. Aus dem eingeengten Extrakt wird das Produkt durch Zusatz von Diäthyläther als violettes Pulver ausgefällt und getrocknet.

Dünnschichtchromatographie System I: $R_f$=0,54.

$\lambda$ max 543 nm (5% Acetonitril).

Beispiel 4

7-(Thienyl-2-acetamido)-3-[2-(4-N-acetoxyäthyl-4-N-cyanoäthyl-amino-phenylazo)-pyridino-methyl]-3-cephem-4-carboxylat

Eine Lösung von 80 mg Cephalostin Na-Salz in 2 ml Wasser wird mit einer Lösung von 45 mg Pyridin-2-azo-4'-N-acetoxyäthyl-N-cyanoäthyl-anilin in der in Beispiel 5 beschriebenen Weise umgesetzt und das Reaktionsprodukt isoliert. Violettes Pulver.

Dünnschichtchromatographie System I: $R_f$=0,54.

$\lambda$ max 520 nm (5% Acetonitril).

Beispiel 5

7-(Thienyl-2-acetamido)-3-[2-(4-N,N-diacetoxyäthylamino-phenylazo)-pyridino-methyl]-3-cephem-4-carboxylat

Eine Lösung von 160 mg Cephalotin Na-Salz in 4 ml Wasser wird mit einer Lösung von 90 mg

Pyridin-2-azo-4'-N,N-diacetoxyäthyl-anilin in der in Beispiel 5 beschriebenen Weise umgesetzt und das Reaktionsprodukt isoliert. Violettes Pulver.

Dünnschichtchromatographie System I: $R_f = 0,64$. λ max 544 nm (5% Acetonitril).

## Beispiel 6

### 7-(Thienyl-2-acetamido)-3-[3-(4-N,N-diäthylamino-phenylazo)-pyridino-methyl]-3-cephem-4-carboxylat

80 mg Cephalotin Na-Salz in 2 ml Wasser werden mit 45 mg Pyridin-3-azo-4'-N,N-diäthylanilin in der in Beispiel 5 beschriebenen Weise umgesetzt und das Reaktionsprodukt isoliert. Rotes Pulver.

Dünnschichtchromatographie System I: $R_f = 0,76$. λ max 506 nm (5% Acetonitril).

## Beispiel 7

### 7-(N-Benzoyl-5-amino-5-carboxy-valeroylamido)-3-[2-(4-N,N-dimethylamino-phenylazo)-pyridino-methyl]-3-cephem-4-carboxylat

13,6 g N-Benzoyl-Cephalosporin C Hexamethylendiamin-Salz werden in 500 ml Wasser gelöst und mit einer Auflösung von 11,3 g PADA in 250 ml Aceton gemischt und auf pH 6,5 gestellt. Unter Rühren wird im Wasserbad 5 Stunden bei 60°C erwärmt, wobei der pH-Wert durch Zugabe von gesättigter Natriumkarbonat-Lösung von 6,5 gehalten wird. Die Umsetzung wird dünnschichtchromatographisch verfolgt (System I). Nach Beendigung der Umsetzung wird die Lösung auf 4°C gekühlt, wobei das nicht umgesetzte PADA fast vollständig ausfällt und nach Abtrennung für neue Umsetzungen verwendet werden kann. Die Lösung wird zur Wasserphase eingeengt (300 ml) und einmal mit 1 Vol. Äthylacetat extrahiert. Nach Einstellung von pH 3 mit n-Schwefelsäure wird die Wasserphase dreimal mit je 1 Vol. Butanol extrahiert, das Butanol im Vakuum auf etwa 60 ml eingeengt und das Reaktionsprodukt durch Zusatz von 2 bis 3 Vol. Diäthyläther ausgefällt. Man erhält 13 g der rohen Titelverbindung als hellviolettes Pulver, das noch beträchtliche Mengen N-Benzoyl-Cephalosporin C enthält.

1,2 g des Rohproduktes werden in 20 ml eines Gemisches von Äthylacetat, Isopropanol und Wasser 4 : 3 : 2 gelöst und auf eine Säule von 100 g Kieselgel »Merck« 60, Korngröße 0,063 – 0,2 mm, im gleichen System eingeschlämmt, aufgetragen und mit dem System entwickelt. Die das gewünschte Produkt enthaltende violette Zone wird getrennt aufgefangen und nach Eindampfen zur Wasserphase gefriergetrocknet. Es werden 68 mg hochgereinigte Titelverbindung als violettes Pulver erhalten.

Dünnschichtchromatogramm System I: $R_f = 0,33$.

λ max 566 nm (5% Acetonitril).

## Beispiel 8

### 7-Amino-3-[2-(4-N,N-dimethylamino-phenylazo)-pyridino-methyl]-3-cephem-4-carboxylat

Eine Auflösung von 1,36 g 7-Aminocephalosporansäure in 50 ml Wasser wird mit einer Lösung von 1,13 g PADA in 25 ml Aceton Vereinigt und auf pH 6,5 eingestellt. Die Umsetzung im Wasserbad von 60°C ist nach 3 Stunden beendet. Nach Abdestillieren des Acetons wird die wäßrige Phase zweimal mit 1 Vol. Äthylacetat extrahiert, auf 15 ml eingedampft und mit 110 ml Aceton versetzt. Das ausgefällte Produkt wird abzentrifugiert, mit Aceton gewaschen und getrocknet. Ausbeute 1,2 g rohe Titelverbindung als violettes Pulver.

200 mg des erhaltenen Rohproduktes werden in 5 ml Wasser gelöst und die Lösung auf eine Säule von 10 ml Adsorptionsharz Diaion HP-20, in Wasser eingeschlämmt, aufgetragen und mit je 50 ml folgender Elutionsmittel entwickelt: Wasser, 10% Acetonitril, 20% Acetonitril. Das gewünschte Produkt wird mit 10%igem und 20%igem Acetonitril eluiert, die aktiven Fraktionenen werden im Vakuum eingedampft, wobei 51 mg hochgereinigte Titelverbindung als violettes Pulver erhalten werden.

Dünnschichtchromatographie System I: $R_f = 0,15$.

λ max 566 nm (5% Acetonitril).

## Beispiel 9

### 7-Phenylacetamido-3-[2-(4-N,N-dimethylamino-phenylazo)-pyridino-methyl]-3-cephem-4-carboxylat

159 mg (0,5 mM) der nach Beispiel 8 erhaltenen 7-Amino-3-[2-(4-N,N-dimethylamino-phenylazo)-pyridino-methyl]-3-cephem-4-carboxylat werden mit 120 mg Natriumkarbonat in 8 ml Wasser und 10 ml Aceton gelöst. Es wird in Eiswasser gekühlt und mit einer Lösung aus 80 mg Phenacetylchlorid in 1 ml Aceton versetzt. Nach einstündigem Rühren bei 0° werden weitere 16 mg Phenacetylchlorid in 0,2 ml Aceton zugesetzt. Nach einer weiteren Stunde bei 0°C wird mit 20 ml $H_2O$ verdünnt, das Aceton im Vakuum entfernt und der pH-Wert auf 8,0 eingestellt. nach zweimaligem Ausschütteln mit Äthylacetat wird die Wasserphase auf pH 1,0 gestellt und so oft mit n-Butanol extrahiert, bis die wäßrige Lösung farblos geworden ist. Die vereinigten Butanolextrakte werden mit der doppelten Menge Chloroform versetzt und zur Trockne eingedampft. Der Rückstand wird zweimal mit Äthanol extrahiert. Nach dem Abdampfen des Äthanols verbleiben 110 mg der Titelverbindung als violettes Pulver mit Phenylessigsäure als Verunreinigung.

Dünnschichtchromatographie System I: $R_f = 0{,}53$.

$\lambda$ max 566 nm (5% Acetonitril).

## Beispiel 10

### 7-(Thienyl-2-acetamido)-3-[4-N,N-dimethylaminophenylazo)-pyridino-methyl]-3-cephem-4-carboxylat (PADAC)

159 mg nach Beispiel 8 erhaltener 7-Amino-3-[2-(4-N,N-dimethylamino-phenylazo)-pyridino-methyl]-3-cephem-4-carboxylat lieferten mit 80 mg Thienyl-2-acetylchlorid analog der in Beispiel 9 beschriebenen Weise 90 mg PADAC, verunreinigt mit Thienylessigsäure.

Dünnschichtchromatographie und $\lambda$ max wie Beispiel 2.

## Beispiel 11

### 7-Phenoxyacetamido-3-[2-(4-N,N-dimethylaminophenylazo)-pyridino-methyl]-3-cephem-4-carboxylat

159 mg nach 8 erhaltener 7-Amino-3-[2-(4-N,N-dimethylamino-phenylazo)-pyridino-methyl]-3-cephem-4-carboxylat lieferten mit 85 mg Phenoxyacetylchlorid analog der in Beispiel 9 beschriebenen Weise 150 mg der violetten Titelverbindung, vereunreinigt durch Phenoxyessigsäure.

Dünnschichtchromatographie System I: $R_f = 0{,}51$.

$\lambda$ max 566 nm (5% Acetonitril).

## Beispiel 12

### 7-(Thienyl-2-acetamido)-3-[2-(4-N-äthyl-4-N-carboxyäthylamino-phenylazo)-pyridino-methyl]-3-cephem-4-carboxylat

400 mg Cephalotin-Natriumsalz werden mit 225 mg Pyridin-2-azo-4'-N-äthyl-4'-N-carboxyäthyl-anilin in der in Beispiel 3 beschriebenen Weise umgesetzt und das Reaktionsprodukt isoliert. Violettes Pulver.

Dünnschichtchromatographie System I: $R_f = 0{,}49$.

$\lambda$ max 566 nm (5% Acetonitril).

Zur Überführung in das leicht wasserlösliche Na-Salz wird das Produkt in Wasser aufgeschlämmt, die Suspension durch vorsichtigen Zusatz von verdünnter NaOH unter Rühren auf pH 7 gestellt und die Lösung gefriergetrocknet.

## Beispiel 13

### 7-(Thienyl-2-acetamido)-3-[3-(4-N-äthyl-4-N-carboxyäthyl-amino-phenylazo)-pyridino-methyl]-3-cephem-4-carboxylat

200 mg Cephalotin-Natriumsalz werden mit 112 mg Pyridin-3-azo-4'-N-äthyl-4'-N-carboxyäthyl-anilin in der in Beispiel 3 beschriebenen Weise umgesetzt und das Reaktionsprodukt isoliert. Rotes Pulver.

Dünnschichtchromatographie System I: $R_f = 0,53$.

$\lambda$ max 499 nm (5% Acetonitril).

Überführung in das Natriumsalz, erfolgt analog Beispiel 12.

Beispiel 14

7-(Thienyl-2-acetamido)-3-[3-(8-N-carboxyäthylamino-chinolin-5-azo)-pyridino-methyl]-3-cephem-4-carboxylat

240 mg Cephalotin Natriumsalz werden mit 180 mg Pyridin-3-azo-5'-(8'-carboxyäthylamino)-chinolin in der in Beispiel 3 beschriebenen Weise umgesetzt und das Reaktionsprodukt isoliert. Rotviolettes Pulver.

Dünnschichtchromatographie System I: $R_f = 0,40$.

$\lambda$ max 516 nm (5% Acetonitril).

Überführung in das wasserlösliche Na-Salz erfolgt analog Beispiel 12.

**Patentansprüche für die Vertragsstaaten: BE, CH, DE, FR, GB, IT, NL, SE**

1. Cephalosporine der allgemeinen Formel I

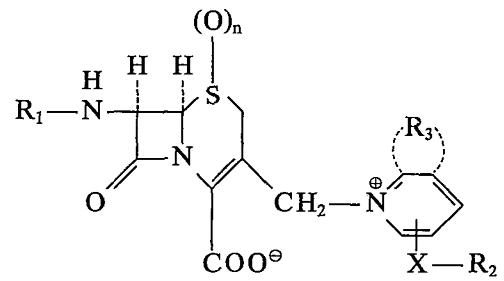

in der

$R_1$ Wasserstoff, Formyl oder einen Rest der Formel

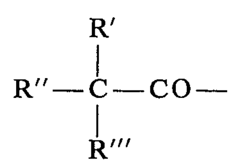

bedeutet, in der R' für Wasserstoff, für Alkyl mit 1 bis 4 C-Atomen, das substituiert sein kann durch Halogen, Cyano, Hydroxy, Alkoxy mit 1 bis 4 C-Atomen, $\omega$-Carboxy, $\omega$-Carboxy-$\omega$-amino oder $\omega$-Carboxy-$\omega$-acylamino mit 1 bis 7 C-Atomen im Acylteil, für einen gesättigten oder ein- oder mehrfach ungesättigten carbocyclischen Ring mit 5 bis 7 C-Atomen, für Phenyl oder Phenyloxy, das ein oder zweifach substituiert sein kann durch Alkyl mit 1 bis 4 C-Atomen, Hydroxy, Alkoxy mit 1 bis 4 C-Atomen, Acyloxy mit 1 bis 4 C-Atomen, Halogen, Carboxy, Sulfoxy, Amino oder Acylamino mit 1 bis 4 C-Atomen, für einen 5- bis 6gliedrigen, heteroaromatischen Ring, der als Heteroatome Stickstoff, Schwefel und/oder Sauerstoff enthalten kann, für einen Thiazolrest der Formel

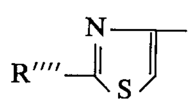

worin R'''' Alkyl mit 1 bis 4 C-Atomen, Amino, Acylamino mit 1 oder 2 C-Atomen, Chlor- Brom- oder Trifluoracetamido bedeutet, für 3,5-Di-chlor-5-pyridon-1-yl, für 2-Methyl-1,3,4-thiadiazol-5-yl-thio oder 2-Amino-1,3,4-thiadiazol-5-yl-thio, R'' und R''', die gleich oder verschieden sein können, für Wasserstoff, Alkyl mit 1 bis 4 C-Atomen, Hydroxy, Acyloxy mit 1 bis 4 C-Atomen, Alkoxy mit 1 bis 4 C-Atomen, Amino, tert.-Butylamino, tert.-Amylamino, Benzylamino, p-Methoxybenzylamino, Benzhydrylamino, Tritylamino, Phenyläthylamino, Formylamino, Acetylamino, Chloracetylamino, Bromacetylamino, Benzoylamino, tert.-Butoxycarbonylamino, 2,2,2-Trichlorethoxycarbonylamino, 4-Hydroxy-1,5-naphthyridin-3-carbonylamino,3-Hydroxy-pyridazin-4-carbonylamino,Imidazolidin-2-on-1-yl-carbonylamino, (3-Methyl-sulfonyl-imidazolidin-2-on-1-yl)-carbonylamino, (4-Ethyl-piperazin-2,3-dion-1-yl)-carbonylamino, Alkoxycarbonyl mit 1 bis 4 C-Atomen im Alkylteil, Halogen, Cyano, Sulfoxy oder Sulfamoyl stehen oder zusammen Sauerstoff sein können,
X für $-(CH=CH)_m-$ mit m = 1 bis 4 oder $-N=N-$ oder Kombinationen dieser Gruppen steht,
$R^2$ für Phenyl steht, das substituiert sein kann durch Hydroxy, Alkoxy mit 1 bis 4 C-Atomen, Acyloxy mit 1 bis 4 C-Atomen oder durch

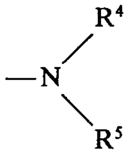

worin $R^4$ und $R^5$ gleich oder verschieden sein können und Wasserstoff, Alkyl mit 1 bis 4 C-Atomen, Cyanoalkyl mit 1 bis 4 C-Atomen im Alkylteil, Carboxyalkyl mit 1 bis 4 C-Atomen im Alkylteil, Alkoxycarbonylalkyl mit jeweils 1 bis 4 C-Atomen in den Alkylteilen, Hydroxyalkyl mit 1 bis 4 C-Atomen, Acyloxyalkyl mit 1 bis 4 C-Atomen im Acyl- und Alkylteil, Carbamylalkyl mit 1 bis 4 C-Atomen im Alkylteil, Sulfoxyalkyl mit 1 bis 4 C-Atomen oder Halogenalkyl mit 1 bis 4 C-Atomen,
$R^3$ für zwei Wasserstoffatome sowie für einen angegliederten Benzolring oder 5- oder 6gliedrigen heteroaromatischen Ring mit 1 bis 2 Stickstoffatomen und/oder einem Schwefelatom steht und
n die Bedeutung von 0, 1 oder 2 besitzt.

2. Verfahren zur Herstellung von Cephalosporinen der allgemeinen Formel I, dadurch gekennzeichnet, daß man eine Verbindung der Formel III

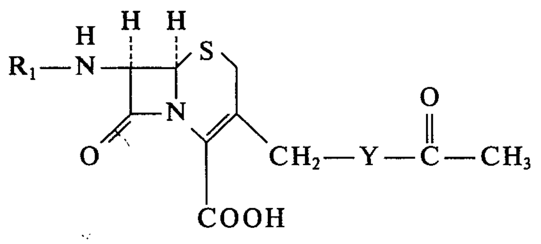

(III)

in der $R_1$ die vorgenannte Bedeutung hat und Y für Sauerstoff oder Schwefel steht, mit einer Verbindung der Formel IV

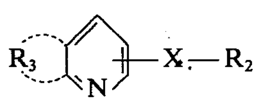

(IV)

in der $R_2$, $R_3$ und X die obengenannten Bedeutungen haben, umsetzt oder daß man Verbindungen der Formel III, in der Y die angegebene Bedeutung besitzt und $R_1$ für Wasserstoff steht, mit einer Verbindung der Formel IV, in der $R_2$, $R_3$ und X die genannten Bedeutungen haben, umsetzt und die erhaltene Verbindung der allgemeinen Formel I, in der $R_1$ für Wasserstoff steht, mit einer Carbonsäure der allgemeinen Formel V

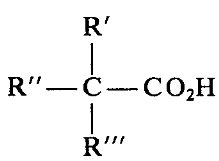

(V)

in der R′, R″ und R‴ die vorgenannten Bedeutungen haben oder einem aktivierten Derivat dieser Carbonsäure zur Umsetzung bringt.

3. Zubereitungen zum Nachweis von $\beta$-Lactamasen, die ein chromophores Cephalosporin der allgemeinen Formel I in Lösung oder auf einer Trägersubstanz enthalten.

4. Zubereitungen gemäß Anspruch 3, in denen die Trägersubstanz Agar darstellt.

5. Zubereitungen gemäß Anspruch 3, in denen die Trägersubstanz Papier (Teststreifen) oder ein anders geeignetes Material darstellt.

6. Verfahren zur Herstellung von Zubereitungen gemäß Ansprüchen 3 bis 5, dadurch gekennzeichnet, daß man ein Cephalosporin der allgemeinen Formel I in einem geeigneten Lösungsmittel löst oder es mit Trägersubstanzen mischt oder auf diese aufbringt.

7. Verwendung von Cephalosporinen der allgemeinen Formel I zum Nachweis von $\beta$-Laxtamasen.

**Patentansprüche für den Vertragsstaat: AT**

1. Verfahren zur Herstellung von Cephalosporinen der allgemeinen Formel I

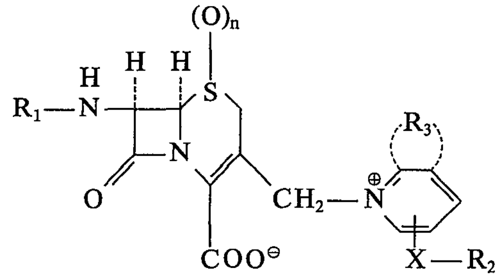

in der

$R_1$ Wasserstoff, Formyl oder einen Rest der Formel

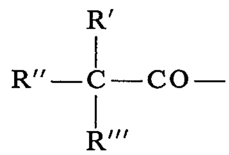

bedeutet, in der R′ für Wasserstoff, für Alkyl mit 1 bis 4 C-Atomen, das substituiert sein kann durch Halogen, Cyano, Hydroxy, Alkoxy mit 1 bis 4 C-Atomen, ω-Carboxy, ω-Carboxy-ω-amino oder ω-Carboxy-ω-acylamino mit 1 bis 7 C-Atomen im Acylteil, für einen gesättigten oder ein- oder mehrfach ungesättigten carbocyclischen Ring mit 5 bis 7 C-Atomen, für Phenyl oder Phenyloxy, das ein oder zweifach substituiert sein kann durch Alkyl mit 1 bis 4 C-Atomen, Hydroxy, Alkoxy mit 1 bis 4 C-Atomen, Acyloxy mit 1 bis 4 C-Atomen, Halogen, Carboxy, Sulfoxy, Amino oder Acylamino mit 1 bis 4 C-Atomen, für einen 5- oder 6gliedrigen, heteroaromatischen Ring, der als Heteroatome Stickstoff, Schwefel und/oder Sauerstoff enthalten kann, für einen Thiazolylrest der Formel

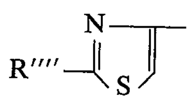

worin

R′′′′ Alkyl mit 1 bis 4 C-Atomen, Amino, Acylamino mit 1 oder 2 C-Atomen, Chlor-, Brom- oder Trifluoracetamido bedeutet, für 3,5-Dichlor-5-pyridon-1-yl, für 2-Methyl-1,3,4-thiadiazol-5-yl-thio oder 2-Amino-1,3-thiadiazol-5-yl-thio, R″ und R‴, die gleich oder verschieden sein können, für Wasserstoff, Alkyl mit 1 bis 4 C-Atomen, Hydroxy, Acyloxy mit 1 bis 4 C-Atomen, Alkoxy mit 1 bis 4 C-Atomen, Amino, tert.-Butylamino, tert.-Amylamino, Benzylamino, p-Methoxybenzylamino, Benzhydrylamino, Tritylamino, Phenylethylamino, Formylamino, Acetylamino, Chloracetylamino, Bromacetylamino, Benzoylamino, tert.-Butoxycarbonylamino, 2,2,2-Trichlorethoxycarbonylamino, 4-Hydroxy-1,5-naphthyridin-3-carbonylamino, 3-Hydroxy-pyridazin-4-carbonylamino, Imidazolidin-2-on-1-yl-carbonylamino, (3-Methyl-sulfonyl- imidazolidin-2-on-1-yl)-carbonylamino, (4-Ethyl-piperazin-2,3-dion-1-yl)-carbonylamino, Alkoxycarbonyl mit 1 bis 4 C-Atomen im Alkylteil, Halogen, Cyano, Sulfoxy oder Sulfamoyl stehen oder zusammen Sauerstoff sein können,

X für $-(CH=CH)_m-$ mit $m=1$ bis 4 oder $-N=N-$ oder Kombinationen dieser Gruppen steht,

$R^2$ für Phenyl steht, das substituiert sein kann und durch Hydroxy, Alkoxy mit 1 bis 4 C-Atomen, Acyloxy mit 1 bis 4 C-Atomen oder durch

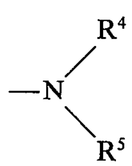

worin $R^4$ und $R^5$ gleich ober verschieden sein können und Wasserstoff, Alkyl mit 1 bis 4 C-Atomen, Cyanoalkyl mit 1 bis 4 C-Atomen im Alkylteil, Carboxyalkyl mit 1 bis 4 C-Atomen im Alkylteil, Alkoxycarbonylalkyl mit jeweils 1 bis 4 C-Atomen in den Alkylteilen, Hydroxyalkyl mit 1 bis 4 C-Atomen, Acyloxyalkyl mit 1 bis 4 C-Atomen im Acyl- und Alkylteil, Carbamoylalkyl mit 1 bis 4 C-Atomen im Alkylteil, Sulfoxyalkyl mit 1 bis 4 C-Atomen oder Halogenalkyl mit 1 bis 4 C-Atomen,

$R^3$ für zwei Wasserstoffatome sowie für einen angegliederten Benzolring oder 5- oder 6gliedrigen heteroaromatischen Ring mit 1 bis 2 Stickstoffatomen und/oder einem Schwefelatom steht und

n die Bedeutung von 0, 1 oder 2 besitzt,
dadurch gekennzeichnet, daß man eine Verbindung der Formel III

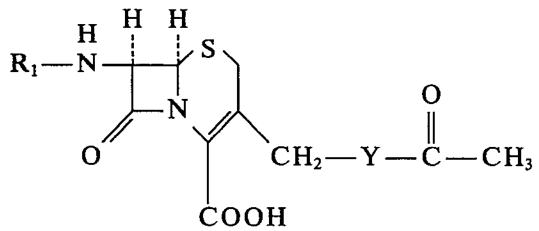

(III)

in der $R_1$ die vorgenannte Bedeutung hat und Y für Sauerstoff oder Schwefel steht, mit einer Verbindung der Formel IV

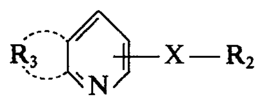

(IV)

in der $R_2$, $R_3$ und X die obengenannten Bedeutungen haben, umsetzt oder daß man Verbindungen der Formel III, in der Y die angegebene Bedeutung besitzt und $R_1$ für Wasserstoff steht, mit einer Verbindung der Formel IV, in der $R_2$, $R_3$ und X die genannten Bedeutungen haben, umsetzt und die erhaltene Verbindung der allgemeinen Formel I, in der $R_1$ für Wasserstoff steht, mit einer Carbonsäure der allgemeinen Formel V

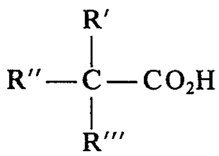

(V)

in der R, R″ und R‴ die vorgenannten Bedeutungen haben oder einem aktivierten Derivat dieser Carbonsäure zur Umsetzung bringt.

2. Verfahren zur Herstellung von Zubereitungen zum Nachweis von β-Lactamasen, dadurch gekennzeichnet, daß man ein Cephalosporin der Formel I in einem geeigneten Lösungsmittel löst oder es mit Trägersubstanzen mischt oder auf diese aufbringt.

3. Verfahren gemäß Anspruch 2, dadurch gekennzeichnet, daß die Trägersubstanz Agar darstellt.

4. Verfahren gemäß Anspruch 3, dadurch gekennzeichnet, daß die Trägersubstanz Papier (Teststreifen) oder ein anderes geeignetes Material darstellt.

5. Verwendung von Cephalosporinen der allgemeinen Formel I zum Nachweis von β-Lactamasen.

**Claims for the contracting states: BE, CH, DE, FR, GB, IT, NL, SE**

1. Cephalosporins of the general formula I

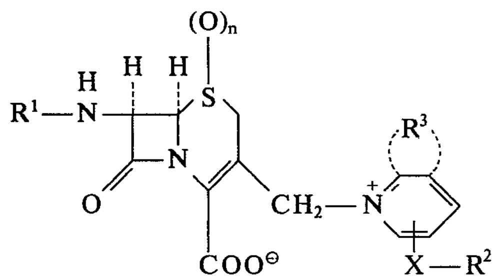

in which
$R^1$ is hydrogen, formyl or a radical of the formula

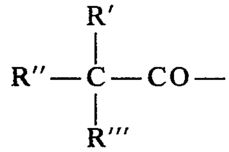

in which R′ is hydrogen, alkyl having 1 to 4 carbon atoms which may be substituted by halogen, by cyano, by hydroxy, by alkoxy with 1 to 4 carbon atoms, by ω-carboxy, ω-carboxy-ω-amino or by ω-carboxy-ω-acylamino with 1 to 7 carbon atoms in the acyl moiety, a saturated or one or several times unsaturated carbocyclic ring with 5 to 7 carbon atoms, phenyl or phenyloxy, which may be substituted once or twice by alkyl with 1 to 4 carbon atoms, by hydroxy, by alkoxy with 1 to 4 carbon atoms, by acyloxy with 1 to 4 carbon atoms, by halogen, by carboxy, by sulfoxy, by amino or by acylamino with 1 to 4 carbon atoms, a 5- to 6-membered heteroaromatic ring, which may contain as hetero atoms nitrogen, sulfur and/or oxygen, a thiazolyl moiety of the formula

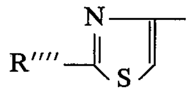

in which R′′′′ denotes alkyl with 1 to 4 carbon atoms, amino, acylamino with 1 or 2 carbon atoms, chloro-, bromo- or trifluoroacetamido, 3,5-dichloro-5-pyridon-1-yl, 2-methyl-1,3,4-thiadiazol-5-yl-thio or 2-amino-1,3,4-thiadiazol-5-yl-thio, R′′ and R′′′ which may be identical or different denote hydrogen, alkyl with 1 to 4 carbon atoms, hydroxy, acyloxy with 1 to 4 carbon atoms, alkoxy with 1 to 4 carbon atoms, amino, tert.-butylamino, tert.amylamino, benzylamino, p-methoxybenzylamino, benzhydrylamino, tritylamino, phenylethylamino, formylamino, acetylamino, chloroacetylamino, bromoacetylamino, benzoylamino, tert.butoxycarbonylamino, 2,2,2-trichloroethoxycarbonylamino, 4-hydroxy-1,5-naphthydrin-3-carbonylamino, 3-hydroxy-pyridazin-4-carbonylamino, imidazolidin-2-on-1-yl-carbonylamino, (3-methyl-sulfonyl)-imidazolidin-2-on-1-yl)-carbonylamino, (4-ethyl-piperazin-2,3-dion-1-yl)-carbonylamino, alkoxycarbonyl with 1 to 4 carbon atoms in the alkyl moiety, halogen, cyano, sulfoxy or sulfamoyl or taken together R′′ und R′′′ denote oxygen,
X is $-(CH=CH)_m-$ with m being 1 to 4, or $-N=N-$ or combinations of said groups,
$R^2$ is phenyl which may be substituted by hydroxy, by alkoxy with 1 to 4 carbon atoms, by acyloxy with 1 to 4 carbon atoms, or by a group of the formula

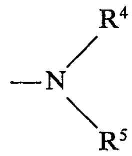

in which $R^4$ and $R^5$ may be identical or different and denote hydrogen, alkyl with 1 to 4 carbon atoms, cyanoalkyl with 1 to 4 carbon atoms in the alkyl moiety, carboxyalkyl with 1 to 4 carbon atoms in the alkyl moiety, alkoxycarbonylalkyl with 1 to 4 carbon atoms in each alkyl moiety, hydroxyalkyl with 1 to 4 carbon atoms, acyloxyalkyl with 1 to 4 carbon atoms in the acyl and alkyl moiety, carbamoylalkyl with 1 to 4 carbon atoms in the alkyl moiety, sulfoxyalkyl with 1 to 4 carbon atoms or haloalkyl with 1 to 4 carbon atoms,
$R^3$ stands for two hydrogen atoms or a connected benzo ring or a connected heteroaromatic 5- or 6-membered ring with 1 to 2 nitrogen atoms and/or one sulfur atom and
n denotes zero 1 or 2, which comprises reacting a compound of the formula III

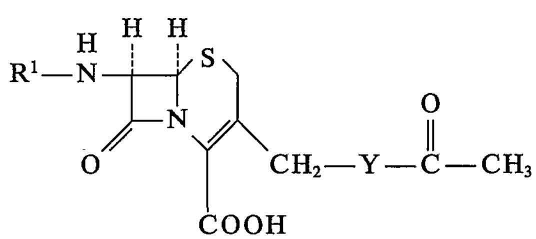

(III)

in which $R^1$ is as defined under formula I and Y denotes oxygen or sulfur with a compound of the formula IV

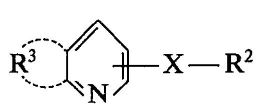

(IV)

in which $R^2$, $R^3$ and X are as defined above, or reacting compounds of the formula III in which Y is as defined above and R′ is hydrogen, with a compounds of the formula IV in which $R^2$, $R^3$ and X are as defined above and reacting the compound of the formula I obtained in which $R^1$ is hydrogen with a carboxylic acid of the formula V

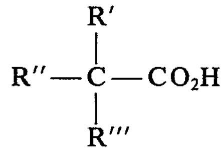

(V)

in which R', R'' and R''' are as defined above or with an activated derivative of said carboxylic acid.

2. Process for making preparations for the detection of $\beta$-Lactamases which comprises dissolving a cephalosporin of the formula I in a suitable solvent, mixing said cephalosporin with a carrier or applying onto a carrier.

3. Process as claimed in claim 2 wherein the carrier is agar-agar.

4. Process as claimed in claim 3, wherein the carrier is paper (test strip) or another suitable material.

5. Method of using cephalosporins of the general formula I for the detection of $\beta$-Lactamases.

**Claims for the contracting state: AT**

1. Process for the manufacture of cephalosporins of the general formula I

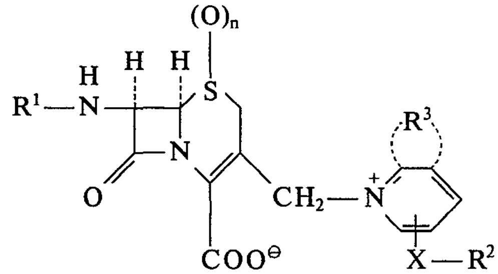

in which

$R^1$ is hydrogen, formyl or a radical of the formula

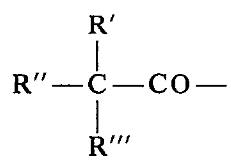

in which R' is hydrogen, alkyl having 1 to 4 carbon atoms which may be substituted by halogen, by cyano, by hydroxy, by alkoxy with 1 to 4 carbon atoms, by $\omega$-carboxy, $\omega$-carboxy-$\omega$-amino, or by $\omega$-carboxy-$\omega$-acylamino with 1 to 7 carbon atoms in the acyl moiety, a saturated or one or several times unsaturated carbocyclic ring with 5 to 7 carbon atoms, phenyl or phenyloxy, which may be substituted once or twice by alkyl with 1 to 4 carbon atoms, by hydroxy, by alkoxy with 1 to 4 carbon atoms, by acyloxy with 1 to 4 carbon atoms, by halogen, by carboxy, by sulfoxy, by amino or by acylamino with 1 to 4 carbon atoms, a 5- to 6-membered heteroaromatic ring, which may contain as hetero atoms nitrogen, sulfur and/or oxygen, a thiazolyl moiety of the formula

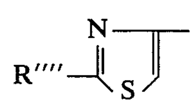

in which R'''' denotes alkyl with 1 to 4 carbon atoms, amino, acylamino with 1 or 2 carbon atoms, chloro-, bromo- or trifluroacetamido, 3,5-dichloro-5-pyridon-1-yl, 2-methyl-1,3,4-thiadiazol-5-yl-thio or 2-amino-1,3,4-thiadiazol-5-yl-thio, R'' and R''' which may be identical or different denote hydrogen, alkyl with 1 to 4 carbon atoms, hydroxy, acyloxy with 1 to 4 carbon atoms, alkoxy with 1 to 4 carbon atoms, amino, tert.butylamino, tert.amylamino, benzylamino, p-methoxybenzylamino, benzhydrylamino, tritylamino, phenylethylamino, formylamino, acetylamino, chloroacetylamino, bromoacetylamino, benzoylamino, tert.butoxycarbonylamino, 2,2,2-trichloroethoxycarbonylamino, 4-hydroxy-1,5-naphthydrin-3-carbonylamino, 3-hydroxy-pyridazin-4-carbonylamino, imidazolidin-2-on-1-yl-carbonylamino, (3-methyl-sulfonyl)-imidazolidin-2-on-1-yl)-carbonylamino, (4-ethyl-piperazin-2,3-dion-1-yl)-carbonylamino, alkoxycarbonyl with 1 to 4 carbon atoms in the alkyl moiety, halogen, cyano, sulfoxy or sulfamoyl or taken together R'' and R''' denote oxygen,

X is $-(CH=CH)_m-$ with m being 1 to 4, or $-N=N-$ or combinations of said groups,

$R^2$ is phenyl which may be substituted by hydroxy, by alkoxy with 1 to 4 carbon atoms, by acyloxy with 1 to 4 carbon atoms, or by a group of the formula

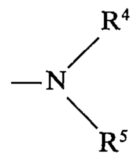

in which $R^4$ and $R^5$ may be identical or different and denote hydrogen, alkyl with 1 to 4 carbon atoms, cyanoalkyl with 1 to 4 carbon atoms in the alkyl moiety, carboxyalkyl with 1 to 4 carbon atoms in the alkyl moiety, alkoxycarbonylalkyl with 1 to 4 carbon atoms in each alkyl moiety, hydroxyalkyl with 1 to 4 carbon atoms, acyloxyalkyl with 1 to 4 carbon atoms in the acyl and alkyl moiety, carbamoylalkyl with 1 to 4 carbon atoms in the alkyl moiety, sulfoxyalkyl with 1 to 4 carbon atoms or haloalkyl with 1 to 4 carbon atoms,
$R^3$ stands for two hydrogen atoms or a connected benzo ring or a connected heteroaromatic 5- or 6-membered ring with 1 to 2 nitrogen atoms and/or one sulfur atom and
n denotes zero, 1 or 2.

2. Process for the manufacture of cephalosporins of the general formula I, which comprises reacting a compound of the formula III

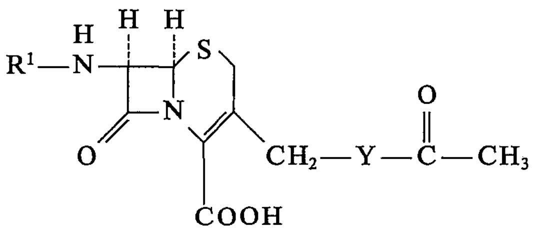

(III)

in which $R^1$ is as defined under formula I and Y denotes oxygen or sulfur with a compound of the formula IV

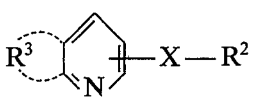

(IV)

in which $R^2$, $R^3$ and X are as defined above, or reacting compounds of the formula III in which Y is as defined above and $R^1$ is hydrogen, with a compound of the formula IV in which $R^2$, $R^3$ and X are as defined above and reacting the compound of the formula I obtained in which $R^1$ is hydrogen with a carboxylic acid of the formula V

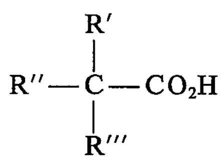

(V)

in which R′, R″ and R‴ are as defined above or with an activated derivative of said carboxylic acid.

3. Preparations for the detection of $\beta$-lactamases containing a chromophoric cephalosporin of the general formula I in solution or on a carrier.

4. Preparations as claimed in claim 3, wherein the carrier is agar-agar.

5. Preparations as claimed in claim 3, wherein the carrier is paper (test strip) or another suitable material.

6. Process for making preparations as claimed in claims 3 to 5, which comprises dissolving a cephalosporin of the general formula I in a suitable solvent, mixing said cephalosporin with a carrier or applying onto a carrier.

7. Method of using cephalosporins of the general formula I for the detection of $\beta$-lactamases.

**Revendications pour les etats contractants: BE, CH, DE, FR, GB, IT, NL, SE**

1. Céphalosporines répondant à la formule générale I:

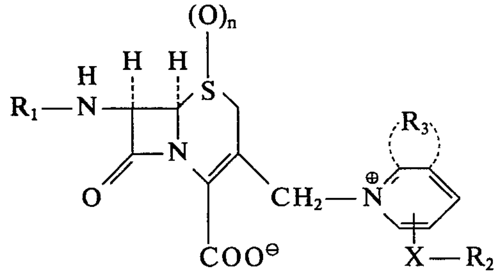

dans laquelle:
$R_1$ représente l'hydrogène, un groupe formyle ou un radical de formule:

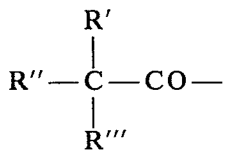

où R′ représente un atome d'hydrogène, un groupe alcoyle ayant de 1 à 4 atomes de carbone, qui peut être substitué par un atome d'halogène, un groupe cyano, hydroxy, alcoxy ayant de 1 à 4 atomes de carbone, ω-carboxy, ω-carboxy-ω-amino ou ω-carboxy-ω-acylamino ayant de 1 à 7 atomes de carbone dans la partie acyle; un noyau carbocyclique saturé ou une ou plusieurs fois insaturé, ayant de 5 à 7 atomes de carbone, un groupe phényle ou phényloxy, qui peut être une ou deux fois substitué par un groupe alcoyle ayant de 1 à 4 atomes de carbone, hydroxy, alcoxy ayant de 1 à 4 atomes de carbon, acyloxy ayant de 1 à 4 atomes de carbone, un atome d'halogène, un groupe carboxy, sulfoxy, amino ou acylamino ayant de 1 à 4 atomes de carbone; un noyau hétéroaromatique ayant de 5 à 6 chainons, qui peut contenir comme hétéroatomes de l'azote, du soufre et/ou de l'oxygène; un radical thiazole de formule:

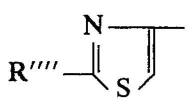

où R′′′′ représente un groupe alcoyle ayant de 1 à 4 atomes de carbone, amino, acylamino avec de 1 à 2 atomes de carbone, le chlore, le brome ou un groupe trifluoroacétamido; un groupe 3,5-di-chloro-5-pyridone-1-yle, un groupe 2-méthyl-1,3,4-thiadiazol-5-yl-thio ou 2-amino-1,3,4-thiadiazol-5-yl-thio; R″ et R‴ qui peuvent être identiques ou différents représentent un atome d'hydrogène, un groupe alcoyle ayant de 1 à 4 atomes de carbone, hydroxy, acyloxy ayant de 1 à 4 atomes de carbone, alcoxy ayant de 1à 4 atomes de carbone, amino, tert-butylamino, tert-amylamino, benzylamino, p-méthoxybenzylamino, benzhydrylamino, tritylamino, phényléthylamino, formylamino, acétylamino, chloracétylamino, bromacétylamino, benzoylamino, tert-butoxycarbonylamino, 2,2,2-trichloroéthoxycarbonylamino, 4-hydroxy-1,5-naphthyridine-3-carbonylamino, 3-hydroxy-pyridazine-4-carbonylamino, imidazolidine-2-one-1-yl-carbonylamino, (3-méthyl-sulfonyl-imidazolidine-2-one-1-yl)-carbonylamino, (4-éthyl-piperazine-2,3-dione-1-yl)-carbonylamino, alcoxy carbonyle avec de 1 à 4 atomes de carbone dans la partie alcoyle, un atome d'halogène, un groupe cyano, sulfoxy ou sulfamoyle, ou peuvent représenter ensemble l'oxygène,
X représente $-(CH=CH)_m-$ avec m = de 1 à 4 ou $-N=N-$ ou une combinaison de ces groupes,
$R^2$ représente un groupe phényle qui peut être substitué par un groupe hydroxy, alcoxy avec de 1 à 4 atomes de carbone, acyloxy avec de 1 à 4 atomes de carbone ou par

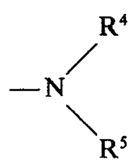

où $R^4$ et $R^5$ peuvent être identiques ou différents et représentent l'hydrogène, un groupe alcoyle ayant de 1 à 4 atomes de carbone, cyanoalcoyle ayant de 1 à 4 atomes de carbone dans la partie alcoyle, carboxyalcoyle ayant de 1 à 4 atomes de carbone dans la partie alcoyle, alcoxycarbonylalcoyle ayant de 1 à 4 atomes de carbone dans chaque partie alcoyle, hydroxyalcoyle ayant de 1 à 4 atomes de

carbone, acyloxyalcoyle ayant de 1 à 4 atomes de carbone dans les parties acyle et alcoyle, carbamoylalcoyle ayant de 1 à 4 atomes de carbone dans la partie alcoyle, sulfoxyalcoyle ayant de 1 à 4 atomes de carbone ou halogènoalcoyle ayant de 1 à 4 atomes de carbone,
$R^3$ représente soit deux atomes d'hydrogène, soit un noyau benzénique condensé ou un noyau hétéroaromatique condensé à 5 ou 6 chainons avec de 1 à 2 atomes d'azote et/ou atome de soufre et n est 0, 1 ou 2.

2. Procédé pour la préparation de céphalosporines de formule générale I, caractérisé en ce qu'on fait réagir un composé de formule III:

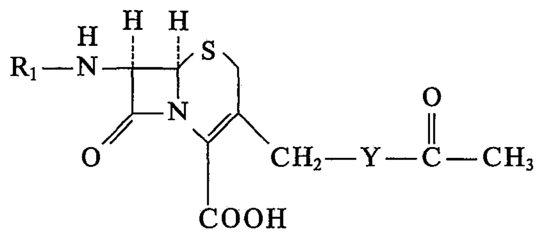

(III)

dans laquelle $R_1$ a la signification indiquée ci-dessus et Y représente l'oxygène ou le soufre, avec un composé de formule IV:

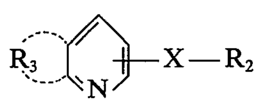

(IV)

dans laquelle $R_2$, $R_3$ et X ont les significations indiquées ci-dessus, ou en ce qu'on fait réagir des composés de formule III où Y a la signification ci-dessus et $R_1$ représente l'hydrogène, avec un composé de formule IV où $R_2$, $R_3$ et X ont les significations indiquées, et on fait réagir le composé de formule générale I obtenu, dans lequel $R_1$ représente l'hydrogène, avec un acide carboxylique de formule générale V:

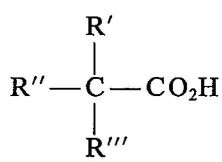

(V)

dans laquelle R′, R″ et R‴ ont les significations indiquées ci-dessus ou avec un dérivé activé de cet acide carboxylique.

3. Compositions pour la détection des $\beta$-lactamases, qui contiennent une céphalosporine chromophore de formule générale I en solution ou sur une substance servant de support.

4. Compisitions selon la revendication 3, dans lesquelles la substance servant de support est la gélose.

5. Compositions selon la revendication 3, dans lesquelles la substance servant de support est du papier (bandes d'essai) ou un autre matériau approprié.

6. Procédé pour la préparation de compositions selon l'une quelconque des revendications 3 à 5, caractérisé en ce qu'on dissout une céphalosporine de formule générale I dans un solvant approprié ou on la mélange avec des substances servant de support ou on l'applique sur ces dernières.

7. Utilisation de céphalosporines de formule générale I pour la détection des $\beta$-lactamases.

**Revendications l'etat contractant: AT**

1. Procédé pour la préparation de céphalosporines de formule générale I:

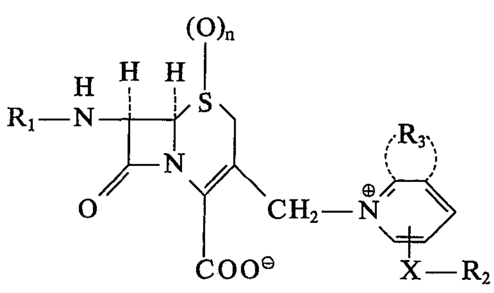

dans laquelle:

$R_1$ représente un atome d'hydrogène, un groupe formyle ou un radical de formule:

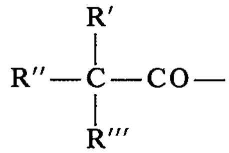

où R′ représente un atome d'hydrogène, un groupe alcoyle ayant de 1 à 4 atomes de carbone, qui peut être substitué par un halogène, un groupe cyano, hydroxy, alcoxy ayant de 1 à 4 atomes de carbone, ω-carboxy, ω-carboxy-ω-amino ou ω-carboxy-ω-acylamino ayant de 1 à 7 atomes de carbone dans la partie acyle; un moyau carbocyclique saturé ou une plusieurs fois insaturé, ayant de 5 à 7 atomes de carbone, un groupe phényloxy, qui peut être une ou deux fois substitué par un groupe alcoyle ayant de 1 à 4 atomes de carbone, hydroxy, alcoxy ayant de 1 à 4 atomes de carbone, acyloxy ayant de 1 à 4 atomes de carbone, un atome d'halogène, un groupe carboxy, sulfoxy, amino ou acylamino ayant de 1 à 4 atomes de carbone; un noyau hétéroaromatique ayant de 5 à 6 chainons, qui peut contenir comme hétéroatomes de l'azote, du soufre et/ou de l'oxygène; un radical thiazole de formule:

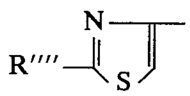

où R′′′′ représente un groupe alcoyle ayant de 1 à 4 atomes de carbone, amino, acylamino ayant de 1 à 2 atomes de carbone, le chlore, le brome ou un groupe trifluoroacétamido; un groupe 3,5-di-chloro-5-pyridone-1-yle, un groupe 2-méthyl-1,3,4-thiadiazol-5-yl-thio ou 2-amino-1,3,4-thiadiazol-5-yl-thio; R′′ et R′′′ qui peuvent être identiques ou différents représentent un atome d'hydrogène, un groupe alcoyle ayant de 1 à 4 atomes de carbone, hydroxy, acyloxy ayant de 1 à 4 atomes de carbone, alcoxy ayant de 1 à 4 atomes de carbone, amino, tert-butylamino, tert-amylamino, benzylamino, p-méthoxybenzylamino, benzhydrylamino, tritylamino, phényléthylamino, formylamino, acétylamino, chloracétylamino, bromacétylamino, benzoylamino, tert-butoxycarbonylamino, 2,2,2-trichloroéthoxycarbonylamino, 4-hydroxy-1,5-naphthyridine-3-carbonylamino, 3-hydroxy-pyridazine-4-carbonylamino, imidazolidine-2-one-1-yl-carbonylamino, (3-méthylsulfonyl-imidazolidine-2-one-1-yl)-carbonylamino, (4-éthyl-pipérazine-2,3-dione-1-yl)-carbonylamino, alcoxyl-carbonyle ayant de 1 à 4 atomes de carbone dans la partie alcoyle, un atome d'halogène, un groupe cyano, sulfoxy ou sulfamoyle ou peuvent représenter ensemble l'oxygène,

X représente $-(CH=CH)_m-$ avec m = de 1 à 4 ou $-N=N-$ ou une combinaison de ces groupes, .

$R_2$ représente un groupe phényle qui peut être substitué par un groupe hydroxy, alcoxy avec de 1 à 4 atomes de carbone, acyloxy avec de 1 à 4 atomes de carbone ou par

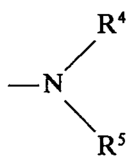

où $R^4$ et $R^5$ peuvent être identiques ou différents et représentent l'hydrogène, un groupe alcoyle ayant de 1 à 4 atomes de carbone, cyanoalcoyle ayant de 1 à 4 atomes de carbone dans la partie alcoyle, carboxyalcoyle ayant de 1 à 4 atomes de carbone dans la partie alcoyle, alcoxy-carbonylalcoyle ayant de 1 à 4 atomes de carbone dans chaque partie alcoyle, hydroxyalcoyle ayant de 1 à 4 atomes de carbone; acyloxyalcoyle ayant de 1 à 4 atomes de carbone dans les parties acyle et alcoyle, carbamoylalcoyle ayant de 1 à 4 atomes de carbone dans la partie alcoyle, sulfoxyalcoyle ayant de 1 à 4 atomes de carbone ou halogéno-alcoyle ayant de 1 à 4 atomes de carbone,

$R^3$ représente soit deux atomes d'hydrogène, soit un noyau benzène condensé ou un noyau hétéroaromatique condensé à 5 ou 6 chainons avec de 1 à 2 atomes d'azote et/ou un atome de soufre, et

n est 0, 1 ou 2;

lequel procédé est caractérisé en ce qu'on fait réagir un composé de formule III:

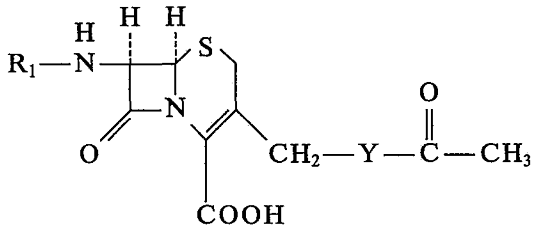

(III)

dans laquelle $R_1$ a la signification précédente et Y représente l'oxygène ou le soufre, avec un composé de formule IV:

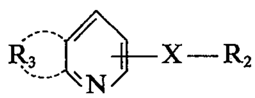

(IV)

dans laquelle $R_2$, $R_3$ et X ont les significations indiquées ci-dessus, ou en ce qu'on fait réagir des composés de formule III dans laquelle Y a la signification indiquée et $R_1$ représente l'hydrogène, avec un composé de formule IV dans laquelle $R_2$, $R_3$ et X ont les significations indiquées, et on fait réagir le composé de formule générale I obtenu, où $R_1$ représente l'hydrogène, avec un acide carboxylique de formule générale V:

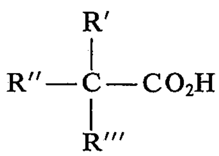

(V)

dans laquelle R', R'' et R''' ont les significations indiquées ci-dessus ou avec un dérivé activé de cet acide carboxylique.

2. Procédé pour la préparation de compositions pour la détection des $\beta$-lactamases, caractérisé en ce qu'on dissout une céphalosporine de formule I dans un solvant approprié ou on la mélange avec des substances servant de support ou on l'applique sur ces dernières.

3. Procédé selon la revendication 2, caractérisé en ce que la substance de support est la gélose.

4. Procédé selon la revendication 3, caractérisé en ce que la substance de support est du papier (bandes d'essai) ou un autre matériau approprié.

5. Utilisation de céphalosporines de formule générale I pour la détection des $\beta$-lactamases.